# EUROPEAN PATENT APPLICATION

(11) **EP 4 484 413 A1**
(43) Date of publication of application: **01.01.2025**
(21) Application number: 23818989.8
(22) Date of filing: 31.05.2023
(51) Int. Cl.: C07D 249/04, C07C 233/05, A61K 9/127, A61K 31/16, A61K 47/18, A61K 45/00

(54) **DENDRON-LIKE LIPID COMPOUND, LIPOSOME, LIPID COMPLEX, AND LIPID NANOPARTICLE AND USE THEREOF**

(30) Priority: 09.06.2022 CN 202210651251
(71) Applicant: Dentarna Therapeutics (Guangzhou) Limited, Guangzhou, Guangdong 510530 (CN)
(72) Inventor: WU, Linping, Guangzhou, Guangdong 510530 (CN); HE, Guantao, Guangzhou, Guangdong 510530 (CN); HUANG, Fujian, Guangzhou, Guangdong 510530 (CN); WANG, Danyang, Guangzhou, Guangdong 510530 (CN); CHEN, Longhui, Guangzhou, Guangdong 510530 (CN)
(74) Representative: Patentwerk B.V.
(86) International application number: PCT/CN2023/097308
(87) International publication number: WO 2023/236824

(57) **Abstract**

The present application provides a dendron-like lipid compound, a liposome, a lipid complex, and a lipid nanoparticle and a use thereof. The dendron-like lipid compound is selected from any one of structures of formulas I-III. The present application provides a series of dendron-like lipid compounds of novel structures; and liposomes prepared from the lipid compounds provided by the present application have uniform particle size distribution, carry negative charges on the surfaces, and have low cytotoxicity. The lipid nanoparticle provided by the present application can specifically target and deliver active substances to cells, tissues or organs, thereby realizing targeted delivery of the active substances.

## Description

### TECHNICAL FIELD

The present application belongs to the technical field of biomedicine, and relates to a drug delivery carrier system, in particular to a dendron-like lipid compound, a liposome, a lipid complex, and a lipid nanoparticle, and a use thereof.

### BACKGROUND

Drug delivery system is very important for the clinical and translational practice of active molecules, especially insoluble small molecules, proteins, and nucleic acid drugs. Nano-delivery system can increase the solubility of active molecules, avoid their degradation, and can specifically target lesions, thereby improving bioavailability and reducing side effects. Therefore, it is of great significance to develop a safe and efficient nano-delivery system. At present, common nano-delivery carrier includes liposomes, dendrimers, polymeric micelles, inorganic nanoparticles, viral vectors, etc.

Lipid nanoparticles (LNP) can not only protect drug molecules from degradation *in vivo* but can also avoid the over-activation of the innate immune system. Furthermore, LNP can promote cellular uptake and specific tissue targeting, thereby reducing the toxic and side effects of drugs and achieving therapeutic effects. Dendron-like lipid is a new type of delivery carrier molecule, the structure of which is mainly composed of a dendron-like head, a lipophilic long-chain tail, and a linker, and is an important class of component constituting novel lipid nanoparticle.

At present, there is a need to develop more new types of lipid compounds to provide more choices for drug delivery.

### SUMMARY

The present application provides a dendron-like lipid compound, a liposome, a lipid complex, and a lipid nanoparticle, and a use thereof. The novel dendron-like lipid molecules for delivery carriers as provided by the present application expand the types of dendron-like lipid compounds. The lipid nanoparticles of the provided novel dendron-like lipid molecules can specifically target and deliver an active substance (such as a small molecule compound, siRNA, mRNA, or a protein, etc.) to a cell, a tissue, or an organ, so as to realize targeted delivery of the active substance.

In a first aspect, the present application provides a dendron-like lipid compound. The dendron-like lipid compound is selected from any one of structures of Formula I to Formula III below: 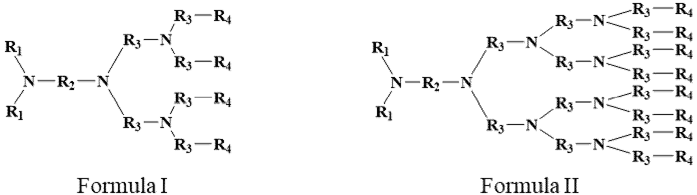 or 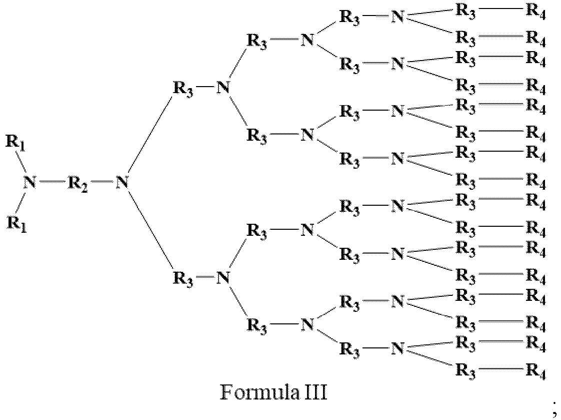
wherein R₁ is selected from 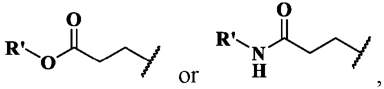 wherein R' is selected from any one of H, C1-C18 (for example, C1, C2, C3, C4, C5, C6, C7, C8, C9, C10, C11, C12, C13, C14, C15, C16, C17, C18, etc.) linear alkyl, or C2-C18 (for example, C2, C3, C4, C5, C6, C7, C8, C9, C10, C11, C12, C13, C14, C15, C16, C17, C18, etc.) linear alkenyl containing 1-3 (for example, 1, 2, 3, etc.) double bonds;
R₂ is selected from 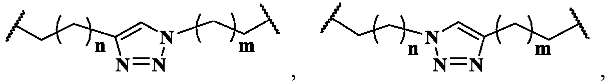 or - (CH₂)ₓ-, wherein m and n are each independently selected from 0 or an integer between 1 and 5 (for example, 1, 2, 3, 4, 5, etc.), and x is selected from an integer between 2 and 8 (for example, 2, 3, 4, 5, 6, 7, 8, etc.);
R₃ is selected from 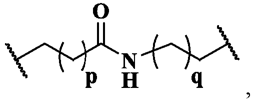 wherein p and q are each independently selected from an integer between 1 and 7 (for example, 1, 2, 3, 4, 5, 6, 7, etc.);
R₄ is selected from 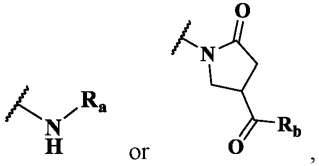 wherein Rₐ is selected from H, substituted or unsubstituted C1-C10 (for example, C1, C2, C3, C4, C5, C6, C7, C8, C9, C10, etc.) acyl, and substituted or unsubstituted benzoyl; and R_{b} is selected from hydroxyl or C1-C10 (for example, C1, C2, C3, C4, C5, C6, C7, C8, C9, C10, etc.) alkoxy; and
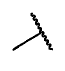 represents a site of attachment in a group.

In the present application, the substituent on the C1-C10 acyl and the substituent on the benzoyl are each independently selected from halogen or C1-C10 (for example, C1, C2, C3, C4, C5, C6, C7, C8, C9, C10, etc.) alkoxy.

Preferably, Rₐ is selected from any one of H, acetyl, propionyl, n-butyryl, isobutyryl, tert-butoxyacyl, trifluoroacetyl, pentafluoropropionyl, heptafluorobutyryl, trichloroacetyl, benzoyl, 4-fluorobenzoyl, 2,3,4,5,6-pentafluorobenzoyl, 4-trifluoromethylbenzoyl, 3,4-dichlorobenzoyl, or 4-trichloromethylbenzoyl.

Preferably, R_{b} is selected from any one of hydroxyl, methoxy, ethoxy, n-propoxy, isopropoxy, or n-butoxy.

In the present application, the dendron-like lipid compound is selected from any one of structures of Formula IV to Formula VI below: 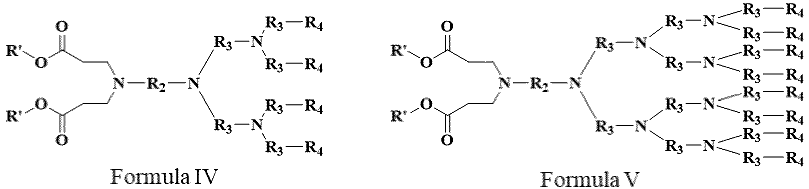 or 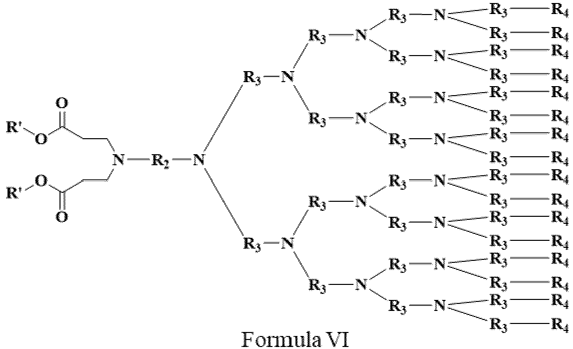
wherein R' is selected from any one of n-octyl, n-dodecyl, n-tridecyl, n-tetradecyl, n-octadecyl, or (9Z,12Z)-n-octadecadienyl, preferably any one of n-dodecyl, n-tridecyl, or (9Z,12Z)-n-octadecadienyl; and
R₂, R₃, and R₄ have the same definitions as those for R₂, R₃, and R₄ in Formula I to Formula III.

Preferably, the dendron-like lipid compound has any one of structures of Formula VII to Formula IX below: 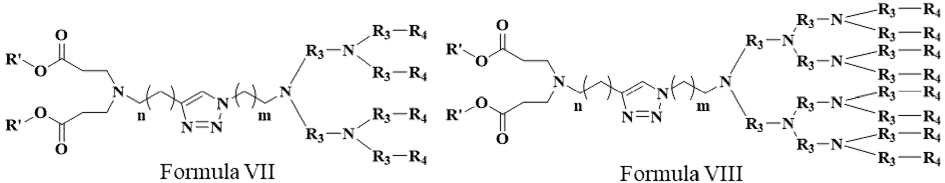 or 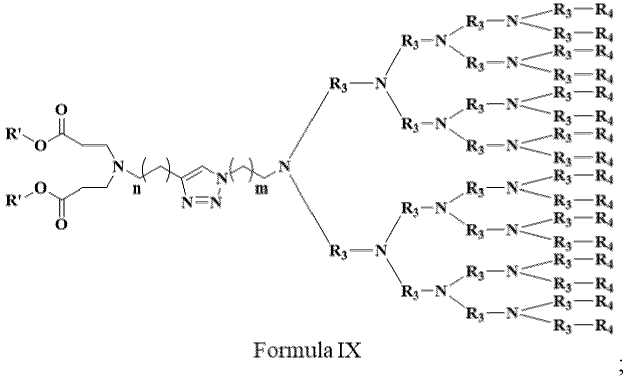
wherein R' is selected from any one of n-octyl, n-dodecyl, n-tridecyl, n-tetradecyl, n-octadecyl, or (9Z,12Z)-n-octadecadienyl, preferably any one of n-dodecyl, n-tridecyl, or (9Z, 12Z)-n-octadecadienyl;
m and n are each independently selected from 0 or an integer between 1 and 5 (for example, 1, 2, 3, 4, 5, etc.); and
R₃ and R₄ have the same definitions as those for R₃ and R₄ in Formula I to Formula III.

In the present application, the dendron-like lipid compound has any one of structures of Formula X to Formula XII below: 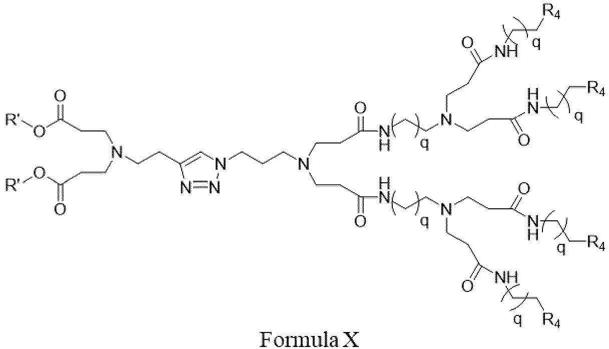 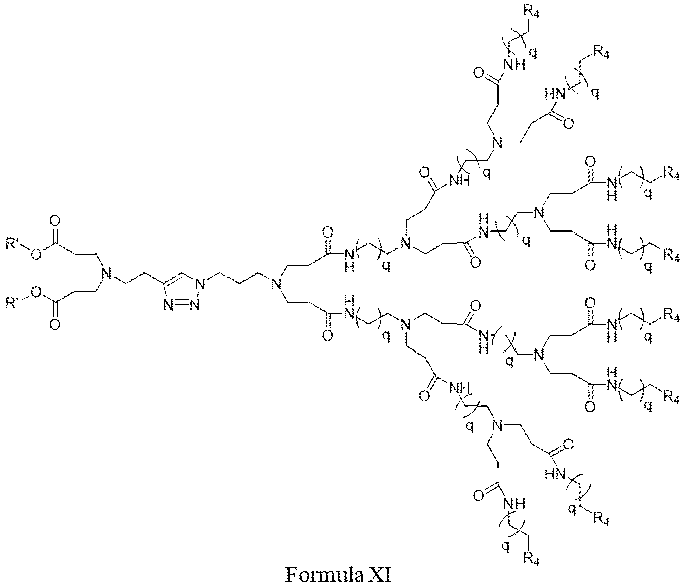 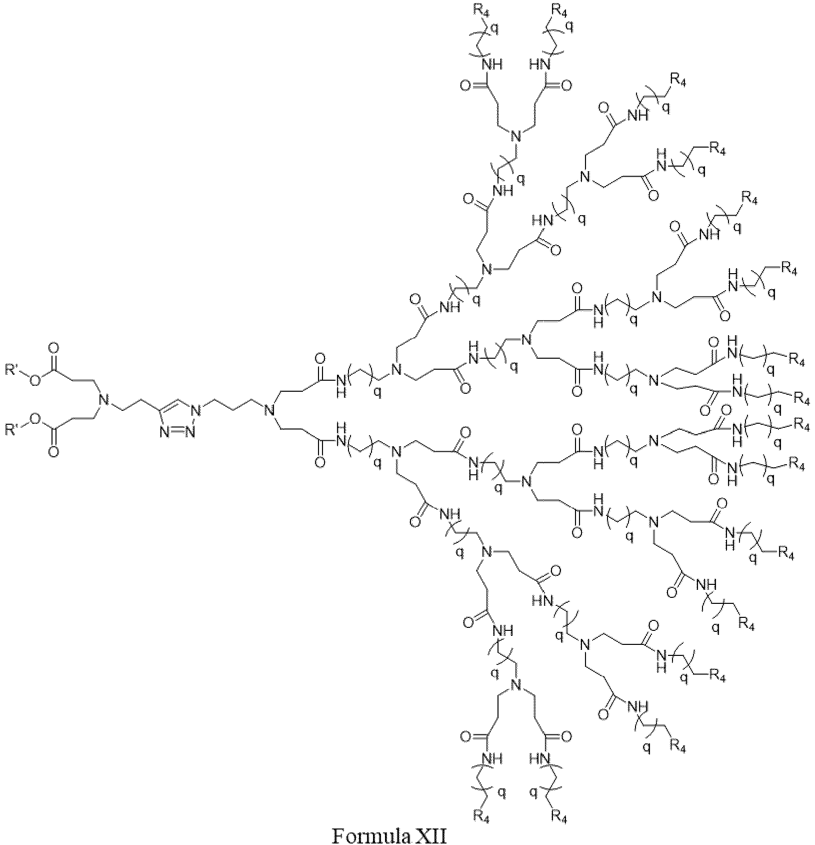 or ;
wherein R' is selected from any one of n-octyl, n-dodecyl, n-tridecyl, n-tetradecyl, n-octadecyl, or (9Z,12Z)-n-octadecadienyl, preferably any one of n-dodecyl, n-tridecyl, or (9Z,12Z)-n-octadecadienyl;
q is selected from an integer between 1 and 7 (for example, 1, 2, 3, 4, 5, 6, 7, etc.); and
R₄ has the same definition as that for R₄ in Formula I to Formula III.

Preferably, the dendron-like lipid compound has any one of structures of Formula XIII to Formula XV below: 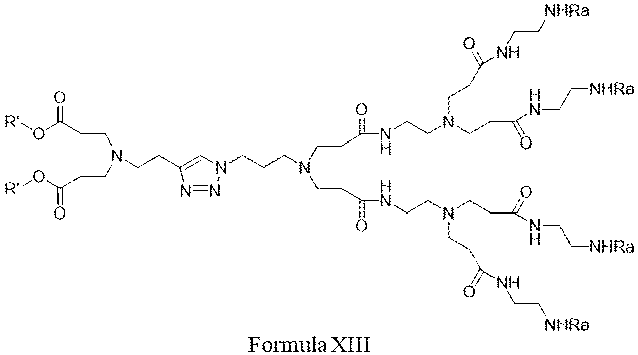 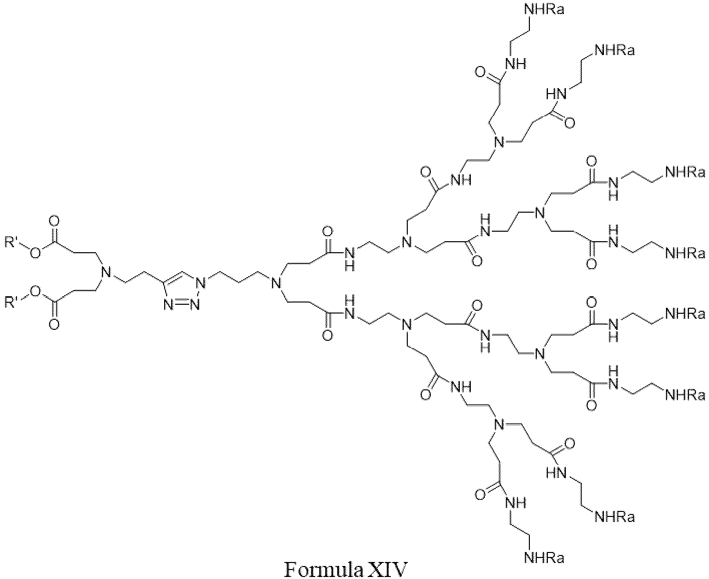 or 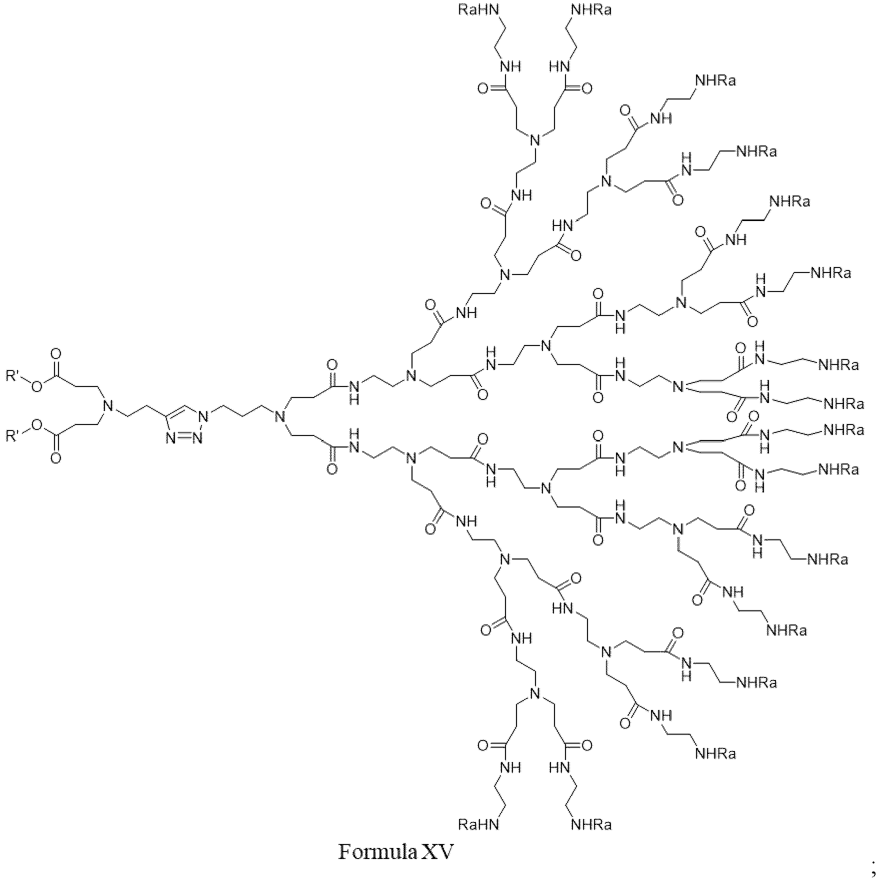 wherein R' is selected from any one of n-octyl, n-dodecyl, n-tridecyl, n-tetradecyl, n-octadecyl, or (9Z,12Z)-n-octadecadienyl, preferably any one of n-dodecyl, n-tridecyl, or (9Z, 12Z)-n-octadecadienyl;

Ra is selected from any one of H, acetyl, propionyl, n-butyryl, isobutyryl, tert-butoxyacyl, trifluoroacetyl, pentafluoropropionyl, heptafluorobutyryl, trichloroacetyl, benzoyl, 4-fluorobenzoyl, 2,3,4,5,6-pentafluorobenzoyl, 4-trifluoromethylbenzoyl, 3,4-dichlorobenzoyl, or 4-trichloromethylbenzoyl; preferably any one of H, tert-butoxyacyl, trifluoroacetyl, pentafluoropropionyl, or heptafluorobutyryl.

Preferably, the dendron-like lipid compound is selected from any one of structures of Formula XVI to Formula XVIII below: 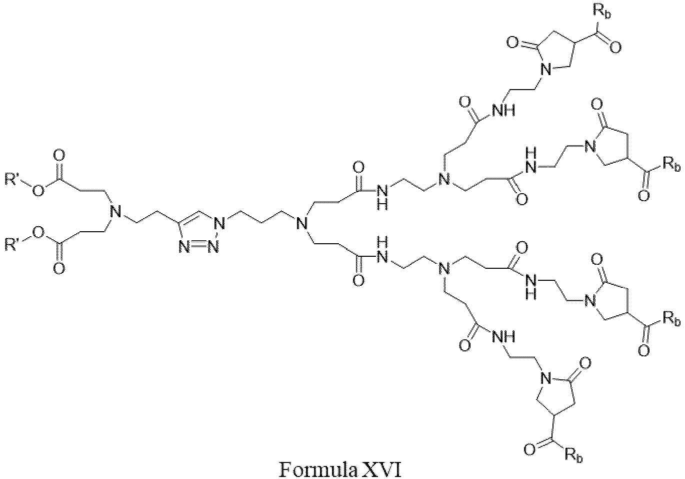 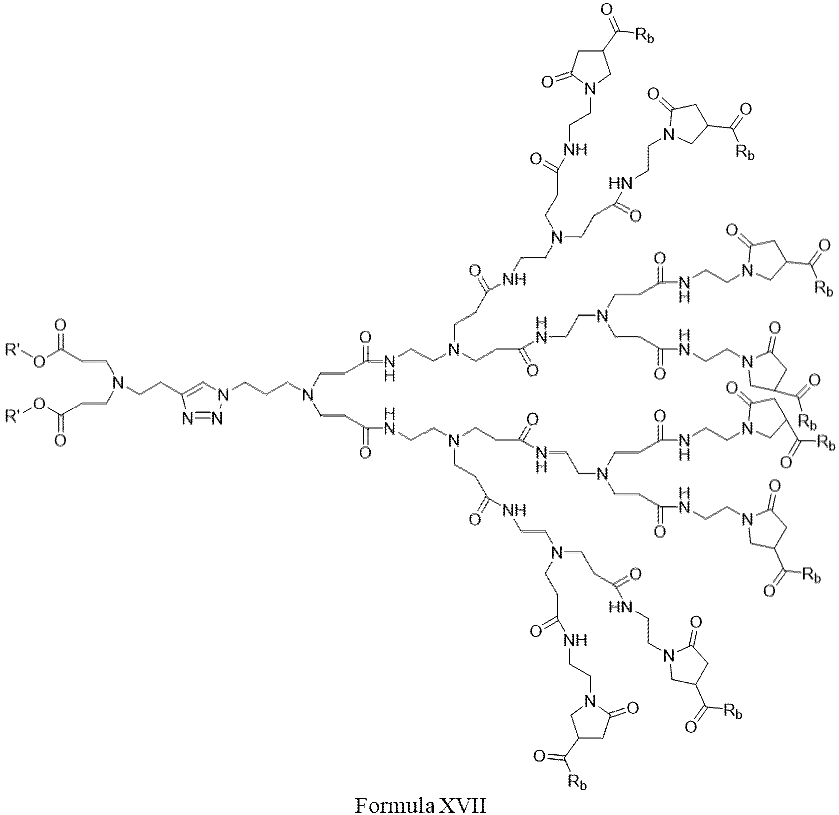 or 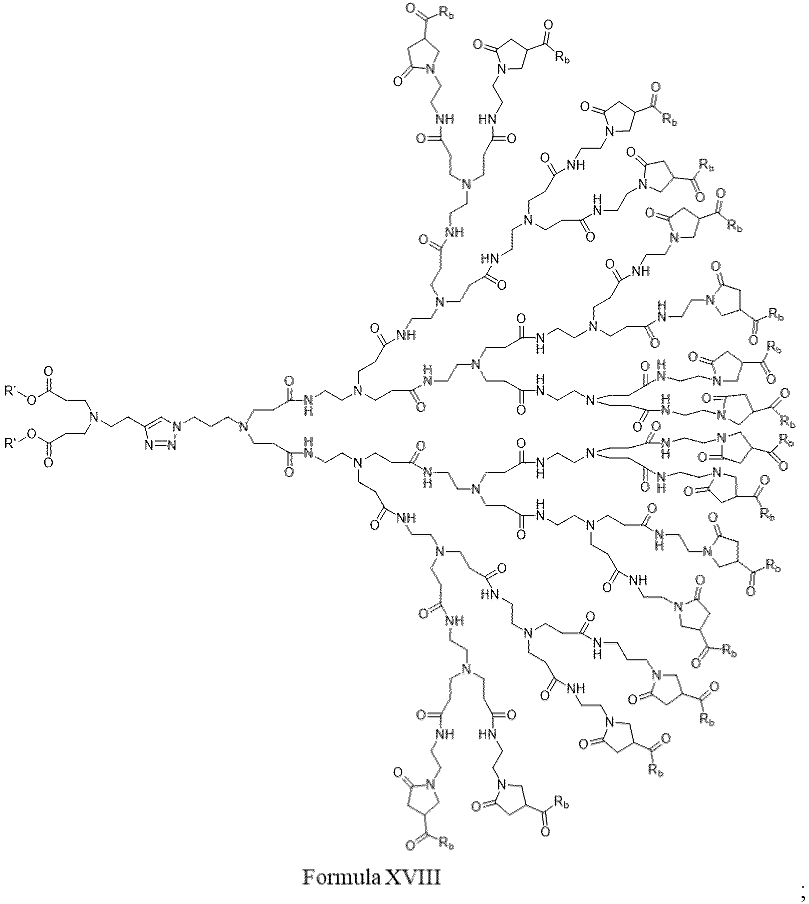
wherein R' is selected from any one of n-octyl, n-dodecyl, n-tridecyl, n-tetradecyl, n-octadecyl, or (9Z,12Z)-n-octadecadienyl, preferably any one of n-dodecyl, n-tridecyl, or (9Z,12Z)-n-octadecadienyl;
R_{b} is selected from any one of hydroxyl, methoxy, ethoxy, n-propoxy, isopropoxy, or n-butoxy, preferably methoxy.

In the present application, the dendron-like lipid compound is selected from any one of the following compounds A1-A21: 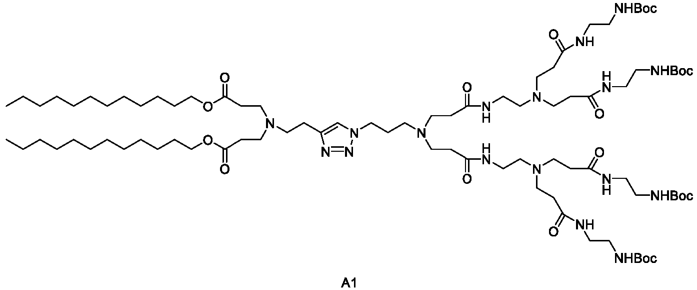 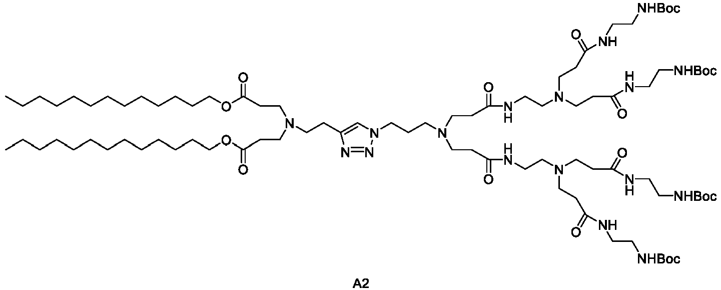 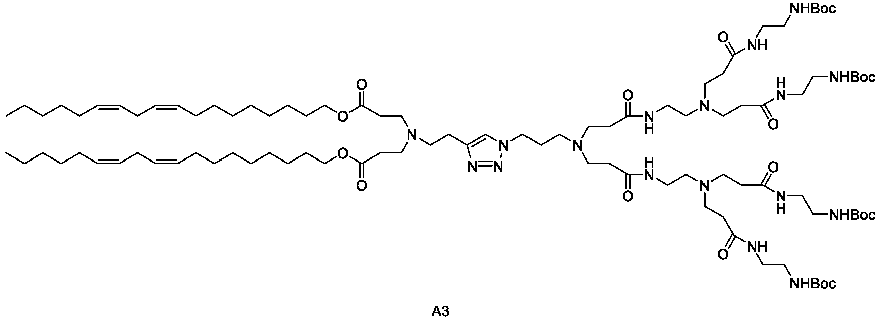 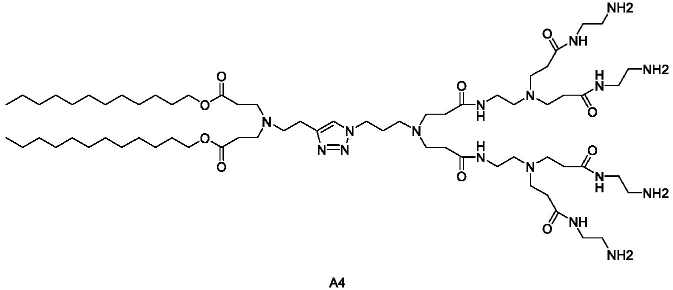 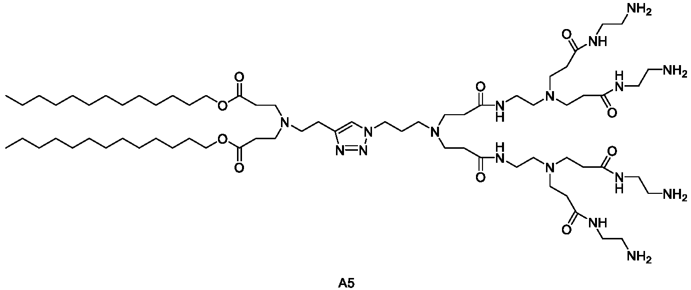 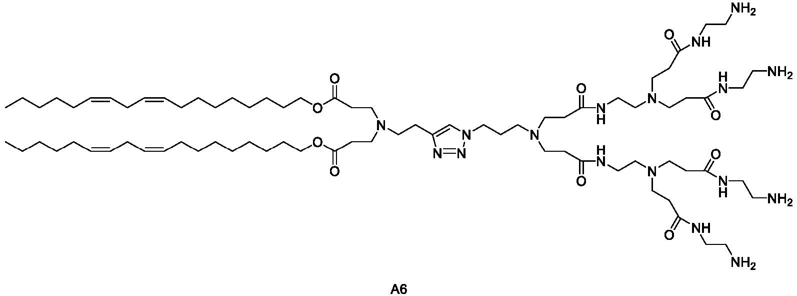 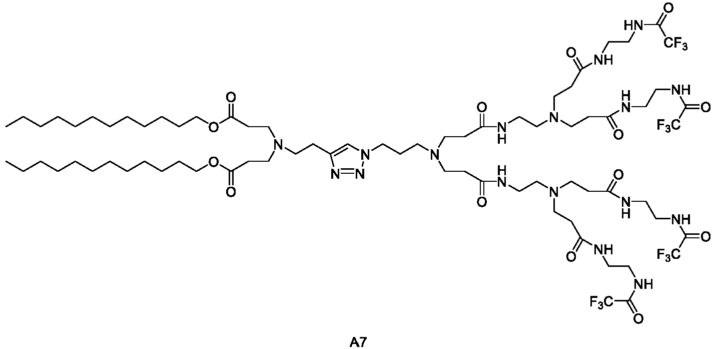 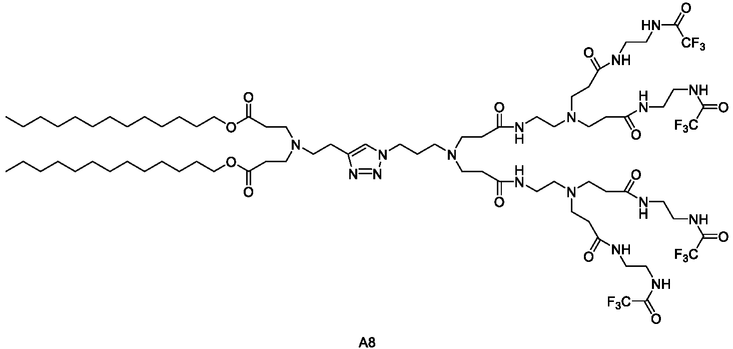 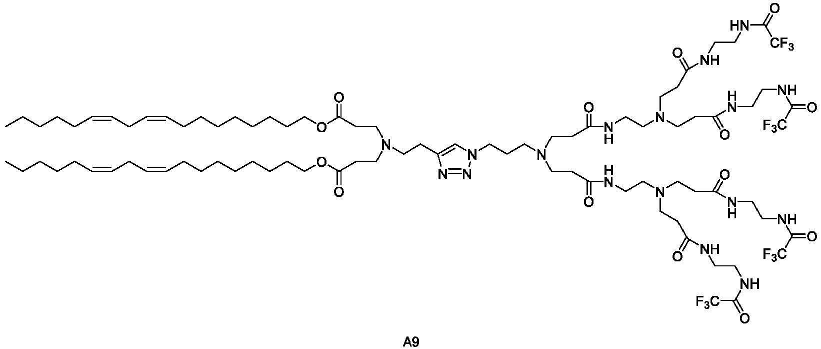 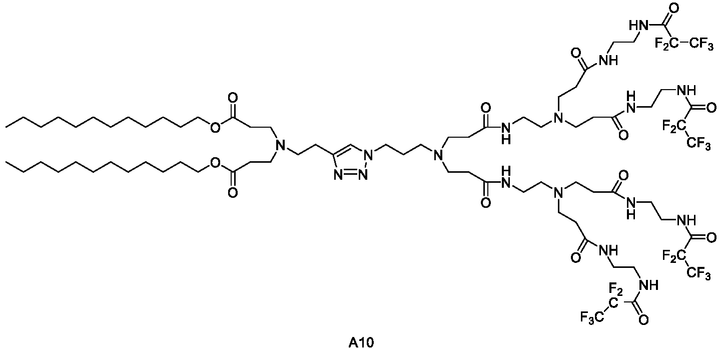 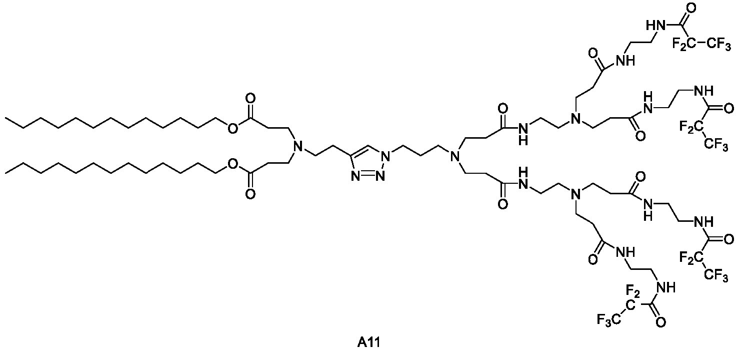 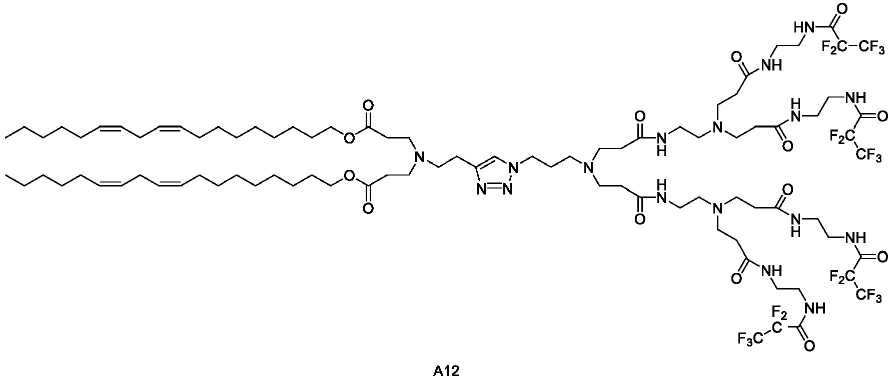 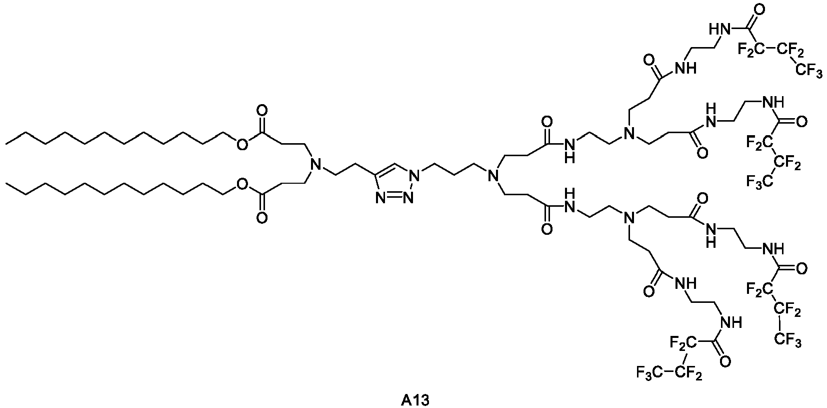 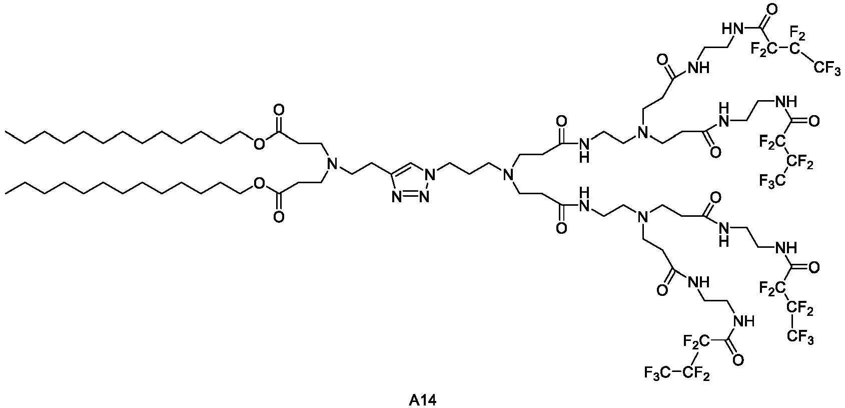 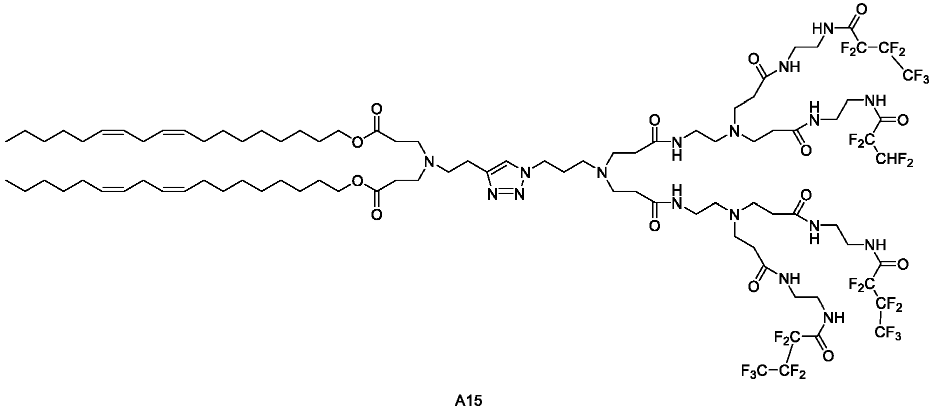 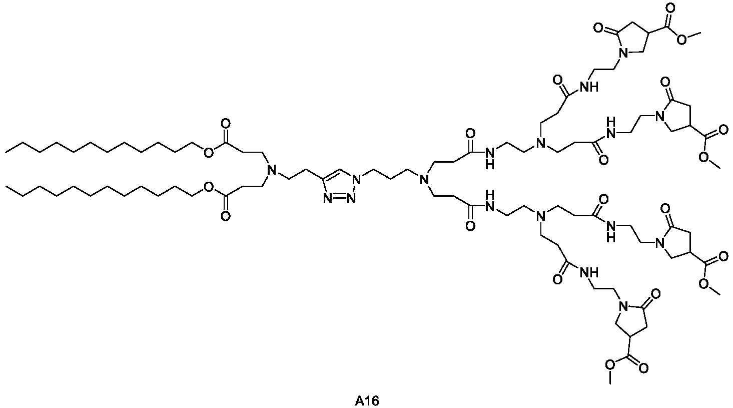 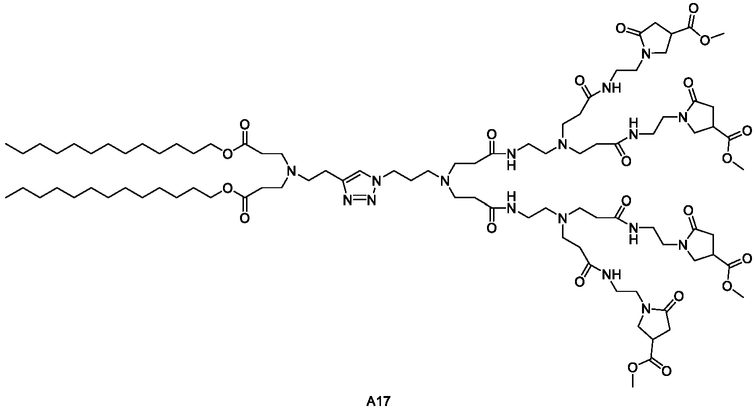 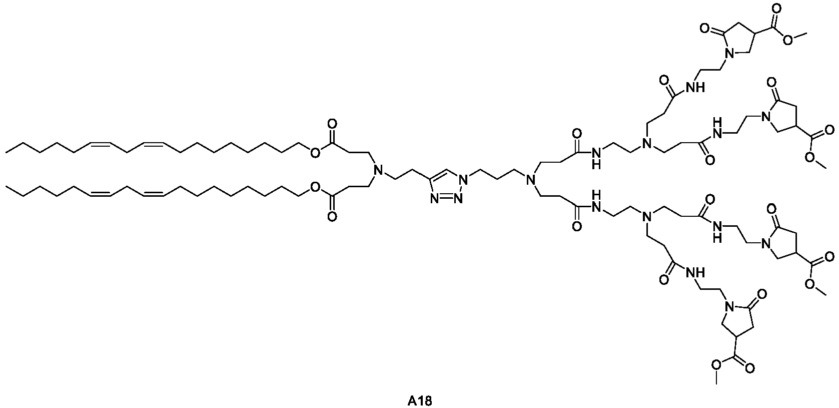 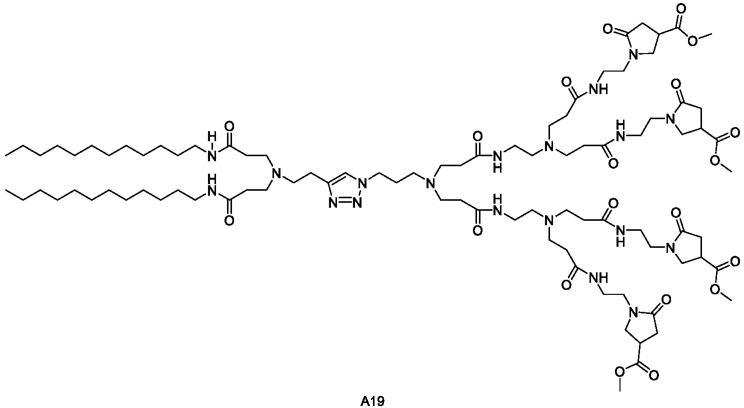 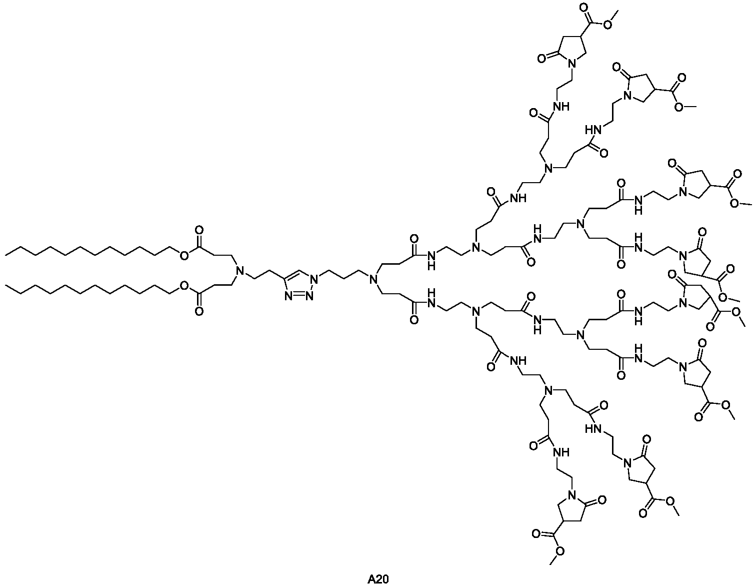 or

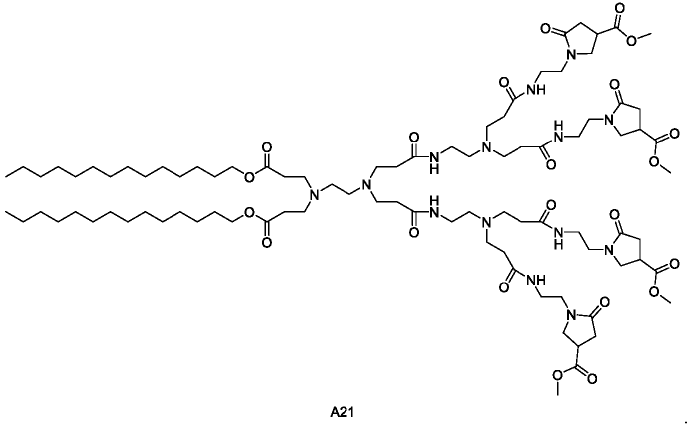

In a second aspect, the present application provides a liposome comprising the dendron-like lipid compound as described in the first aspect.

Preferably, the liposome further comprises an auxiliary lipid.

Preferably, the auxiliary lipid comprises a phospholipid.

Preferably, the phosphatide comprises any one of or a combination of at least two of 1,2-dilinoleoyl-sn-glycero-3-phosphocholine (DLPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-3-phosphocholine (DUPC), 1-palmitoyl-2-oleoyl-sn-glycero-3-phosphocholine (POPC), 1,2-di-O-octadecenyl-sn-glycero-3-phosphocholine (18ODiether PC), 1-oleoyl-2-cholesterylhemisuccinoylsn-glycero-3-phosphocholine (OChemsPC), 1-cetyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine (DOPE), 1,2-didocosahexaenoyl-sn-glycero-3-phosphoethanolamine, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoyl-phosphatidyl-ethanolamine (DSPE), dipalmitoylphosphatidylethanolamine (DPPE), dimyristoylphosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, or lysophosphatidylethanolamine (LPE).

Preferably, the auxiliary lipid further comprises a structured lipid.

Preferably, the structured lipid comprises at least any at least one of or a combination of at least two of cholesterol, coprosterol, sitosterol, ergosterol, rapeseed sterol, soybean sterol, rapeseed sterol, stigmasterol, brassicasterol, tomatidine, ursolic acid, or α-tocopherol.

Preferably, the auxiliary lipid further comprises a PEGylated lipid.

Preferably, the PEGylated lipid comprises any one of or a combination of at least two of PEG-modified phosphatidylethanolamine, PEG-modified phosphatidic acid, PEG-modified ceramide, or PEG-modified dialkylamine.

Preferably, the liposome comprises the dendron-like lipid compound, the phospholipid, the structured lipid, and the PEGylated lipid.

Preferably, the liposome comprises, by mole percentage, 20%-80% (for example, 20%, 30%, 40%, 50%, 60%, 70%, 80%, etc.) of a dendron-like lipid compound, 5%-30% (for example, 5%, 7%, 9%, 11%, 13%, 15%, 17%, 19%, 21%, 23%, 25%, 27%, 29%, 30%, etc.) of a phospholipid, 10%-50% (for example, 10%, 20%, 30%, 40%, 50%, etc.) of a structured lipid, and 1%-10% (for example, 1%, 2%, 3%, 4%, 5%, 6%, 7%, 8%, 9%, 10%, etc.) of a PEGylated lipid.

In a third aspect, the present application provides a lipid complex, wherein the lipid complex comprises the liposome described in the second aspect and an active substance.

Preferably, the active substance comprises any one of or a combination of at least two of a small molecule compound, nucleic acid molecules, or protein molecules.

Preferably, the nucleic acid molecules comprise any at least one of or a combination of at least two of DNA, mRNA, siRNA, aiRNA, miRNA, dsRNA, aRNA, or lncRNA.

Preferably, the protein molecules comprise any one or a combination of at least two of an enzyme, an antibody, a short peptide, a polypeptide, or a recombinant protein.

Preferably, a method for preparing the lipid complex comprises a step of mixing a liposome with an active substance by means of a mixer to obtain the lipid complex.

In a fourth aspect, the present application provides a lipid nanoparticle, wherein the lipid nanoparticle comprises the liposome described in the second aspect, or the lipid complex described in the third aspect.

Preferably, the particle size of the lipid nanoparticle is 40-300 nm (for example, 40 nm, 50 nm, 60 nm, 70 nm, 80 nm, 90 nm, 100 nm, 120 nm, 140 nm, 160 nm, 180 nm, 200 nm, 220 nm, 240 nm, 260 nm, 280 nm, 300 nm, etc.).

In a fifth aspect, the present application provides a reagent, kit, preparation or pharmaceutical composition, wherein the reagent, kit, preparation or pharmaceutical composition each independently comprises any one of or a combination of at least two of the dendron-like lipid compound described in the first aspect, the liposome described in the second aspect, the lipid complex described in the third aspect, or the lipid nanoparticle described in the fourth aspect.

In a sixth aspect, the present application provides a use of the dendron-like lipid compound according to the first aspect, the liposome according to the second aspect, the lipid complex according to the third aspect, the lipid nanoparticle according to the fourth aspect, or the reagent, kit, preparation, or pharmaceutical composition according to the fifth aspect in the manufacture of a product with any one of the following functions (1)-(4):
(1) encapsulating an active substance;
(2) delivering an encapsulated active substance to a cell, a tissue, or an organ;
(3) enabling a delivered active substance to function in a cell, a tissue, or an organ; and
(4) preventing, diagnosing or treating a disease.

Compared with the prior art, the present application has the following beneficial effects:
The present application provides a series of dendron-like lipid compounds with novel structures. Liposomes prepared from a lipid compound provided by the present application have a uniform particle size distribution, carry negative charges on the surface, and have a low cytotoxicity. The lipid nanoparticle provided by the present application can specifically target and deliver an active substance (such as a small molecule compound, siRNA, mRNA, or a protein, etc.) to a cell, a tissue, or an organ, thereby realizing targeted delivery of the active substance.

### BRIEF DESCRIPTION OF DRAWINGS

FIG. 1a shows the uptake of liposomes by RAW264.7 macrophages.
FIG. 1b shows the uptake of liposomes by A549 cancer cells.
FIG. 1c shows the uptake of different liposomes by macrophages and cancer cells as verified by flow cytometry.
FIG. 2a is a graph showing the toxicity of liposomes A13-A15 to RAW264.7 macrophages.
FIG. 2b is a graph showing the toxicity of liposomes A13-A15 to A549 cancer cells.
FIG. 2c is a graph showing the toxicity of liposomes A16-A18 to RAW264.7 macrophages.
FIG. 2d is a graph showing the toxicity of liposomes A16-A18 to A549 cancer cells.
FIG. 3 shows siRNA transfection in eGFR-231 cells by different dendron-like liposomes.
FIG. 4 shows mRNA transfection in A549 cells by different dendron-like liposomes.
FIG. 5a shows images of fluorescent liposome A9 in animals upon organ imaging.
FIG. 5b shows the fluorescence intensity of fluorescent liposome A9 in animals upon organ imaging.
FIG. 5c shows images of fluorescent liposome A18 in animals upon organ imaging.
FIG. 5d shows the fluorescence intensity of fluorescent liposome A18 in animals upon organ imaging.

### DETAILED DESCRIPTION

The technical solution of the present application will be further demonstrated below through specific embodiments. It should be clear to those skilled in the art that the examples only assist in understanding the present application and should not be regarded as specific limitations to the present application.

The sources of products in the following examples and application examples are as follows:

| Product | Manufacturer | Trade mark |
|---|---|---|
| siRNA | Guangzhou RiboBio Co., Ltd. | siN0000001-1-5 siRNC #1, 5 nmol |
| mRNA | Shanghai Hongene Biotechnology Limited | ON-243 EGFP mRNA |
| RAW264.7 cells | Wuhan Pricella Biotechnology Co., Ltd. | CL-0190 |
| A549 cells | Shanghai EK-Bioscience Biotechnology Co., Ltd. | CC-Y1035 |
| BALB/C mice | Zhuhai BesTest Biotechnology Co., Ltd. | bst-05-2 |

### EXAMPLE 1

This example provided dendron-like lipid compound A1. A method for preparing the dendron-like lipid compound A1 included the following steps:

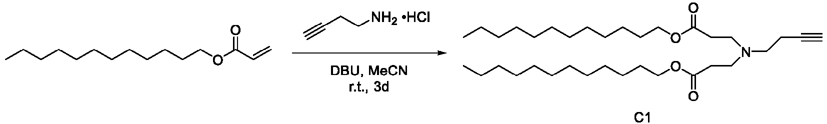

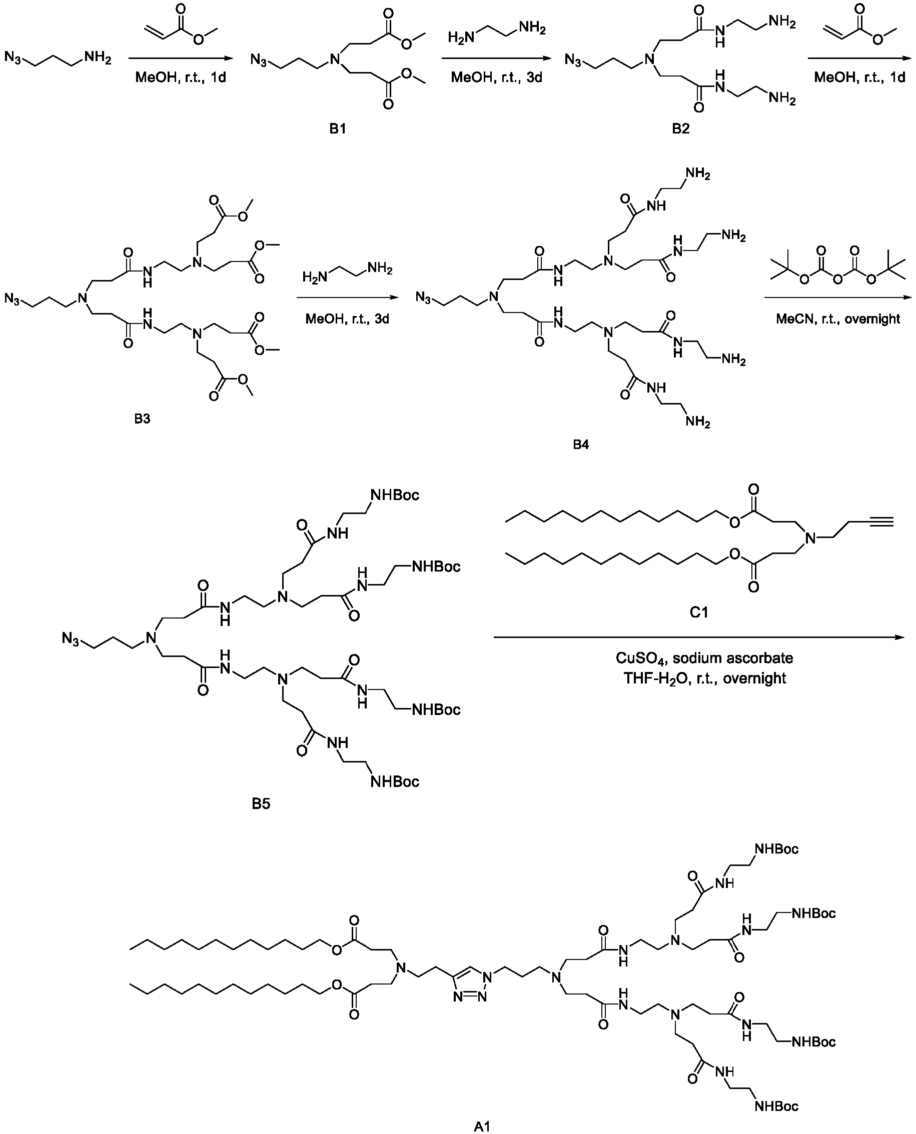

### (1) Synthesis of intermediate B1

Under argon protection, 3-azidopropylamine (0.5 g, 4.99 mmol) was dissolved in 4.2 mL of anhydrous methanol and stirred in an ice-water bath, and 4.2 mL of anhydrous methanol dissolved with methyl acrylate (1.14 g, 13.24 mmol) was slowly added dropwise within 1 h. After the addition was completed, the reaction was kept at a low temperature for 1 h, the ice-water bath was then removed, and the reaction was continued at room temperature for 1 day. The resulted reaction product was concentrated under reduced pressure to dryness, and separated and purified by silica gel column [petroleum ether : ethyl acetate = 5 : 1 (V/V)] to obtain colorless oil B1 with a yield of 80%.

MS: *m*/*z* (M+H⁺) 272.7. ¹H NMR (500 MHz, CDCl₃) δ ppm: 3.66 (s, 6H), 3.30 (t, *J*=1.0Hz, 2H), 2.73 (t, J=1.5Hz, 4H), 2.48 - 2.41 (m, 6H), 1.71 - 1.65 (m, 2H).

### (2) Synthesis of intermediate B3

Under argon protection, ethylenediamine (5 mL, 74.9 mmol) was dissolved in 4.8 mL of anhydrous methanol and stirred in an ice-water bath, and 4.8 mL of anhydrous methanol dissolved with B1 (1.0 g, 3.67 mmol) was slowly added dropwise within 1 h. After the addition was completed, the reaction was kept at a low temperature for 1 h, the ice-water bath was then removed, and the reaction was continued at room temperature for 3 days. After concentration under reduced pressure to dryness, 20 mL of a toluene-methanol mixed solution [toluene : methanol = 9 : 1 (V/V)] was added, and the ethylenediamine remaining after the reaction was removed by azeotropic distillation under reduced pressure. 20 mL of methanol was added, and toluene was removed by azeotropic distillation under reduced pressure until it was dry, thus obtaining crude yellow oil B2. The crude product B2 could be directly used in the next reaction.

Under argon protection, crude B2 (1.1 g, 3.35 mmol) was dissolved in 17 mL of anhydrous methanol and stirred in an ice-water bath, and 17 mL of anhydrous methanol dissolved with methyl acrylate (2.2 mL, 24.4 mmol) was slowly added dropwise within 1 h. After the addition was completed, the reaction was kept at a low temperature for 1 h, the ice-water bath was then removed, and the reaction was continued at room temperature for 1 day. The resulted reaction product was concentrated under reduced pressure to dryness, and separated and purified by silica gel column [petroleum ether : ethyl acetate = 5 : 1 (V/V)] to obtain colorless oil B3 with a yield of 60%.

MS: *m*/*z* (M+H⁺) 673.4. ¹H NMR (500 MHz, Chloroform-*d*) δ ppm: 6.99 (t, *J =* 5.1 Hz, 2H), 3.70 (s, 12H), 3.35 - 3.28 (m, 6H), 2.81 - 2.76 (m, 12H), 2.58 - 2.52 (m, 6H), 2.45 (t, *J=* 6.7 Hz, 8H), 2.38 (t, *J=* 6.7 Hz, 4H), 1.75 (d, *J=* 6.7 Hz, 2H).

### (3) Synthesis of intermediate B5

Under argon protection, ethylenediamine (12 mL, 179.7 mmol) was dissolved in 12 mL of anhydrous methanol and stirred in an ice-water bath, and 12 mL of anhydrous methanol dissolved with B3 (2 g, 2.97 mmol) was slowly added dropwise within 1 h. After the addition was completed, the reaction was kept at a low temperature for 1 h, the ice-water bath was then removed, and the reaction was continued at room temperature for 3 days. After concentration under reduced pressure to dryness, 20 mL of a toluene-methanol mixed solution [toluene : methanol = 9 : 1 (V/V)] was added, and the ethylenediamine remaining after the reaction was removed by azeotropic distillation under reduced pressure. 20 mL of methanol was added, and toluene was removed by azeotropic distillation under reduced pressure until it was dry. The operation of azeotropic distillation under reduced pressure was repeated twice to obtain crude yellow oil B4. The crude product B4 could be directly used in the next reaction.

Under argon protection, crude B4 (1.0 g, 1.27 mmol) was dissolved in 8 mL of anhydrous acetonitrile and stirred in an ice-water bath, and Boc anhydride (2.78 g, 12.7 mmol) was added. The reaction was kept at a low temperature for 1 h, the ice-water bath was then removed, and the reaction continued at room temperature overnight. The resulted reaction product was concentrated under reduced pressure to dryness, and separated and purified by silica gel column [dichloromethane : methanol = 1 : 1 (V/V)] to obtain light yellow oil B5 with a yield of 82%.

MS: *m*/*z* (M+H⁺) 1185.9. ¹H NMR (500 MHz, Chloroform-*d*) δ ppm: 7.63 (s, 2H), 7.47 (s, 4H), 5.66 (s, 4H), 3.35 - 3.18 (m, 22H), 2.70 (q, *J=* 8.1, 7.1 Hz, 12H), 2.51 (q, *J=* 7.5, 6.9 Hz, 6H), 2.34 (t, *J=* 6.2 Hz, 12H), 1.69 (p, *J=* 6.8 Hz, 2H), 1.41 (s, 36H).

### (4) Synthesis of intermediate C1

Under argon protection, 3-butyne-1-amine hydrochloride (0.5 g, 4.74 mmol) was dissolved in 8 mL of anhydrous acetonitrile, and DBU (0.72 g, 4.74 mmol) and dodecyl acrylate (5.69 g, 23.7 mmol) were added and reacted for 3 days at room temperature. The resulted reaction product was concentrated under reduced pressure to dryness, and separated and purified by silica gel column [petroleum ether : ethyl acetate = 20 : 1 (V/V)] to obtain light yellow oil C1 with a yield of 76%.

MS: *m*/*z* (M+H⁺) 550.3. ¹H NMR (500 MHz, Chloroform-*d*) δ ppm: 4.06 (t, *J =* 6.8 Hz, 4H), 2.83 (t, *J =* 7.2 Hz, 4H), 2.70 (t, *J =* 7.5 Hz, 2H), 2.46 (t, *J =* 7.2 Hz, 4H), 2.31 (td, *J =* 7.5, 2.7 Hz, 2H), 1.96 (t, *J=* 2.7 Hz, 1H), 1.69 - 1.58 (m, 6H), 1.43 - 1.16 (m, 40H), 0.88 (t, *J=* 6.8 Hz, 6H).

### (5) Synthesis of compound A1

Under argon protection, B5 (119 mg, 0.10 mmol) and C1 (66 mg, 0.12 mmol) were dissolved in 2 mL of tetrahydrofuran, and copper sulfate pentahydrate (15 mg, 0.06 mmol) and sodium L-ascorbate (24 mg, 0.12 mmol) were added and dissolved by adding 1 mL of water and reacted overnight at room temperature. The reaction product was concentrated under reduced pressure, freeze-dried to remove water, and separated and purified by silica gel column [dichloromethane : methanol = 4: 1 (V/V)] to obtain white solid A1 with a yield of 78%.

MS: *m*/*z* [(M+2H⁺)/2] 869.1. ¹H NMR (500 MHz, Methanol-*d*₄) δ ppm: 7.81 (s, 1H), 4.39 (t, *J=* 7.1 Hz, 2H), 4.07 (t, *J=* 6.6 Hz, 4H), 3.27 (dt, *J=* 11.9, 6.4 Hz, 12H), 3.17 (t, *J =* 6.2 Hz, 8H), 2.88 - 2.72 (m, 20H), 2.58 (t, *J =* 6.7 Hz, 4H), 2.47 (q, *J =* 7.1 Hz, 6H), 2.35 (q, *J =* 6.4 Hz, 12H), 2.07 (*q, J =* 6.8 Hz, 2H), 1.63 (p, *J* = 6.9 Hz, 4H), 1.43 (s, 36H), 1.30 (d, *J =* 7.7 Hz, 40H), 0.90 (t, *J =* 6.8 Hz, 6H).

### EXAMPLE 2

This example provided dendron-like lipid compound A2. A method for preparing the dendron-like lipid compound A2 included the following steps:

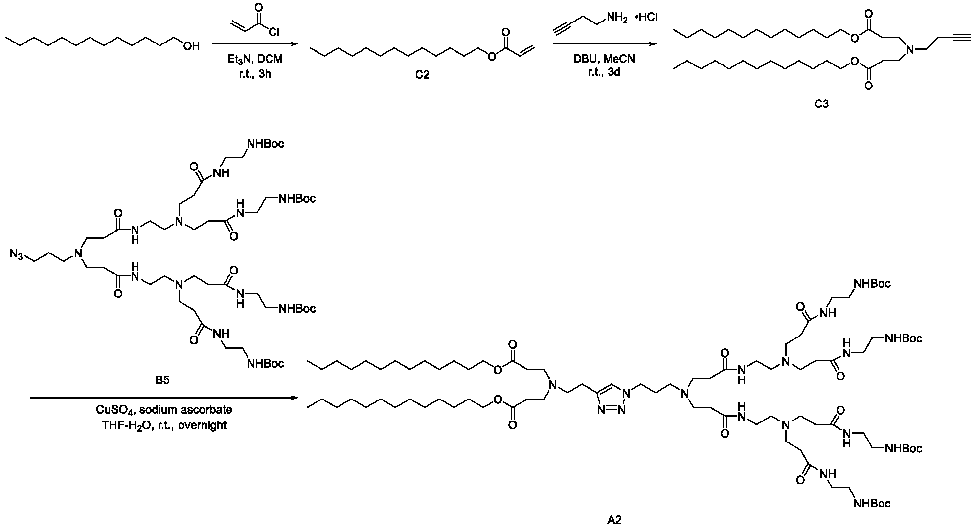

### (1) Synthesis of intermediate C2

Under argon protection, tridecanol (1 g, 4.99 mmol) was dissolved in 6 mL of anhydrous dichloromethane and stirred in an ice-water bath, triethylamine (0.7 mL, 4.99 mmol) was added, and acryloyl chloride (0.68 g, 7.49 mmol) was slowly added dropwise within 0.5 h. After the addition was completed, the ice-water bath was then removed, and the reaction was continued at room temperature for 3 h. 10 mL of dichloromethane and 10 mL of water were added and fully stirred, the organic phase was separated, washed with 10 mL of a saturated sodium chloride aqueous solution, and dried over anhydrous sodium sulfate. The resulted reaction product was concentrated under reduced pressure to dryness, and separated and purified by silica gel column [petroleum ether : ethyl acetate = 30 : 1 (V/V)] to obtain colorless oil C2 with a yield of 90%.

¹H NMR (400 MHz, Chloroform-d) δ ppm: 6.42 (dd, *J=* 17.3, 1.6 Hz, 1H), 6.14 (dd, *J=* 17.3, 10.4 Hz, 1H), 5.83 (dd, *J =* 10.4, 1.6 Hz, 1H), 4.17 (t, *J=* 6.7 Hz, 2H), 1.69 (p, *J* = 6.8 Hz, 2H), 1.49 - 1.17 (m, 22H), 0.90 (t, *J =* 6.7 Hz, 3H).

### (2) Synthesis of intermediate C3

Referring to the method in step (4) of Example 1, light yellow oil C3 was synthesized from C2 as a starting material, with a yield of 70%.

MS: *m*/*z* (M+H⁺) 578.4. ¹H NMR (400 MHz, Chloroform-*d*) δ ppm: 4.08 (t, *J =* 6.8 Hz, 4H), 2.83 (t, *J =* 7.2 Hz, 4H), 2.71 (t, *J =* 7.5 Hz, 2H), 2.47 (t, *J =* 7.1 Hz, 4H), 2.33 (td, *J =* 7.5, 2.7 Hz, 2H), 1.96 (t, *J=* 2.7 Hz, 1H), 1.65 (q, *J=* 6.9 Hz, 4H), 1.37 - 1.24 (m, 40H), 0.91 (t, *J* = 6.7 Hz, 6H).

### (3) Synthesis of compound A2

Referring to the method in step (5) of Example 1, light yellow oil A2 was synthesized from B5 and C3 as starting materials, with a yield of 75%.

MS: *m*/*z* [(M+2H⁺)/2] 882.6. ¹H NMR (500 MHz, Methanol-*d*₄) δ ppm: 7.81 (s, 1H), 4.39 (t, *J=* 7.1 Hz, 2H), 4.06 (t, *J=* 6.6 Hz, 4H), 3.27 (dt, *J=* 11.9, 6.4 Hz, 12H), 3.15 (t, *J=* 6.2 Hz, 8H), 2.88 - 2.72 (m, 20H), 2.58 (t, *J =* 6.7 Hz, 4H), 2.47 (q, *J =* 7.1 Hz, 6H), 2.35 (q, *J =* 6.4 Hz, 12H), 2.05 (q, *J=* 6.8 Hz, 2H), 1.63 (p, *J=* 6.9 Hz, 4H), 1.43 (s, 36H), 1.30 (d, *J=* 7.7 Hz, 44H), 0.90 (t, *J =* 6.8 Hz, 6H).

### EXAMPLE 3

This example provided dendron-like lipid compound A3. A method for preparing the dendron-like lipid compound A3 included the following steps:

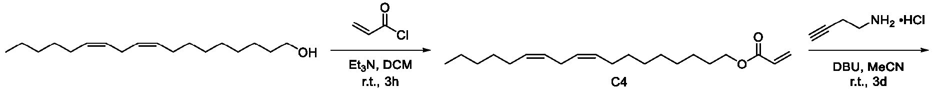

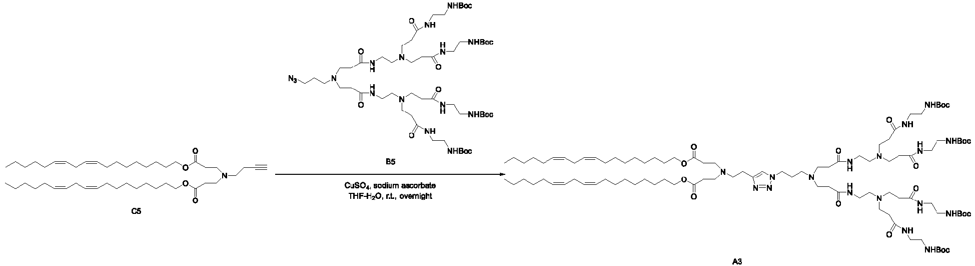

### (1) Synthesis of intermediate C4

Referring to the method in step (1) of Example 2, light yellow oil C4 was synthesized from linoleny alcohol as a starting material, with a yield of 86%.

MS: *m*/*z* (M+Na⁺) 343.5. ¹H NMR (400 MHz, Chloroform-*d*) δ ppm: 6.42 (dd, *J =* 17.4, 1.6 Hz, 1H), 6.15 (dd, *J =* 17.4, 10.4 Hz, 1H), 5.83 (dd, *J =* 10.4, 1.6 Hz, 1H), 5.38 (qd, *J =* 11.4, 9.8, 4.0 Hz, 4H), 4.18 (t, *J =* 6.7 Hz, 2H), 2.80 (t, *J =* 6.4 Hz, 2H), 2.08 (q, *J =* 6.8 Hz, 4H), 1.73 - 1.66 (m, 2H), 1.43 - 1.32 (m, 16H), 0.92 (t, *J=* 6.7 Hz, 3H).

### (2) Synthesis of intermediate C5

Referring to the method in step (4) of Example 1, light yellow oil C5 was synthesized from C4 as a starting material, with a yield of 66%.

MS: *m*/*z* (M+H⁺) 710.2. ¹H NMR (500 MHz, Chloroform-*d*) δ ppm: 5.44 - 5.30 (m, 8H), 4.08 (t, *J =* 6.8 Hz, 4H), 2.81 (dt, *J =* 15.8, 7.0 Hz, 8H), 2.70 (t, *J =* 7.5 Hz, 2H), 2.47 (t, *J =* 7.2 Hz, 4H), 2.33 (td, *J =* 7.5, 2.7 Hz, 2H), 2.07 (q, *J =* 7.0 Hz, 8H), 1.96 (t, *J =* 2.7 Hz, 1H), 1.64 (p, *J =* 6.9 Hz, 6H), 1.38 - 1.30 (m, 30H), 0.91 (t, *J =* 6.8 Hz, 6H).

### (3) Synthesis of compound A3

Referring to the method in step (5) of Example 1, light yellow oil A3 was synthesized from B5 and C5 as starting materials, with a yield of 72%.

MS: *m*/*z* [(M+2H⁺)/2] 948.9. ¹H NMR (500 MHz, Methanol-*d*₄) δ ppm: 7.80 (s,1H), 5.42 - 5.26 (m, *J* = 6.9, 5.8 Hz, 8H), 4.39 (t, *J=* 7.1 Hz, 2H), 4.05 (t, *J=* 6.7 Hz, 4H), 3.34 (s, 6H), 3.29 - 3.11 (m, 20H), 2.80 (ddq, *J =* 23.5, 12.3, 6.4 Hz, 24H), 2.60 (t, *J =* 6.6 Hz, 4H), 2.48 (dt, *J=* 13.8, 6.6 Hz, 6H), 2.36 (q, *J=* 6.5 Hz, 12H), 2.06 (q, *J=* 6.9 Hz, 10H), 1.63 (p, *J=* 6.8 Hz, 4H), 1.42 (s, 36H), 1.33 (tdd, *J=* 15.8, 10.5, 5.5 Hz, 36H), 0.90 (t, *J=* 6.8 Hz, 6H).

### EXAMPLE 4

This example provided dendron-like lipid compound A4. A method for preparing the dendron-like lipid compound A4 included the following steps:

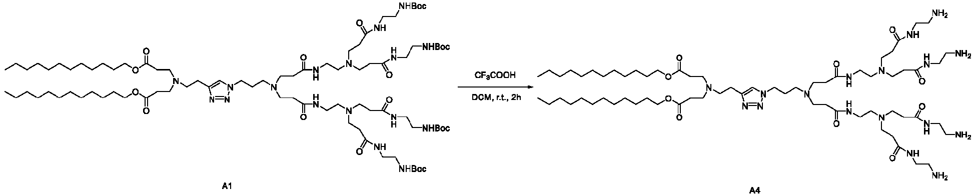

Compound A1 (35 mg, 0.02 mmol) was dissolved in 1 mL of anhydrous dichloromethane, and 0.5 mL of trifluoroacetic acid was added and reacted for 2 h at room temperature. After concentration under reduced pressure, an appropriate amount of water was added, the pH was adjusted to 10 with a saturated sodium bicarbonate aqueous solution, and after back extraction with an appropriate amount of dichloromethane, the aqueous phase was taken and freeze-dried. After dissolution by adding an appropriate amount of anhydrous ethanol, filtration, and separation and purification by Sephadex-G-25 gel column, white oil A4 was obtained with a yield of 77%.

MS: *m*/*z* [(M+2H⁺)/2] 668.2. ¹H NMR (500 MHz, Methanol-*d*₄) δ ppm:7.82 (s, 1H), 4.39 (t, *J =* 7.0 Hz, 2H), 4.05 (t, *J =* 6.6 Hz, 4H), 3.34 (d, *J =* 9.1 Hz, 8H), 3.27 (t, *J =* 6.8 Hz, 4H), 2.80 (dp, *J=* 26.3, 6.5, 6.0 Hz, 24H), 2.58 (t, *J=* 6.8 Hz, 4H), 2.46 (t, *J=* 6.7 Hz, 6H), 2.36 (dt, *J =* 13.5, 6.8 Hz, 12H), 2.04 (t, *J =* 6.9 Hz, 2H), 1.63 (p, *J =* 6.7 Hz, 4H), 1.30 (d, *J =* 6.8 Hz, 40H), 0.90 (t, *J =* 6.8 Hz, 6H).

### EXAMPLE 5

This example provided dendron-like lipid compound A5. A method for preparing the dendron-like lipid compound A5 included the following steps:

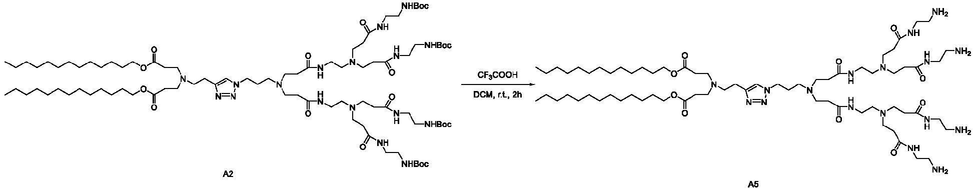

Referring to the method in Example 4, white oil C5 was synthesized from A2 as a starting material, with a yield of 74%.

MS: *m*/*z* [(M+2H⁺)/2] 682.6. ¹H NMR (500 MHz, Methanol-*d*₄) δ ppm:7.84 (s, 1H), 4.41 (t, *J=* 7.1 Hz, 2H), 4.07 (t, *J=* 6.6 Hz, 4H), 3.37 (s, 10H), 3.29 (t, *J=* 6.9 Hz, 4H), 2.81 (ddd, *J =* 26.4, 13.1, 6.2 Hz, 24H), 2.60 (t, *J =* 6.8 Hz, 4H), 2.48 (t, *J =* 6.6 Hz, 6H), 2.38 (dt, *J =* 14.7, 6.7 Hz, 12H), 2.09 - 2.04 (m, 2H), 1.65 (t, *J=* 7.1 Hz, 4H), 1.31 (s, 44H), 0.92 (d, *J=* 6.6 Hz, 6H).

### EXAMPLE 6

This example provided dendron-like lipid compound A6. A method for preparing the dendron-like lipid compound A6 included the following steps:

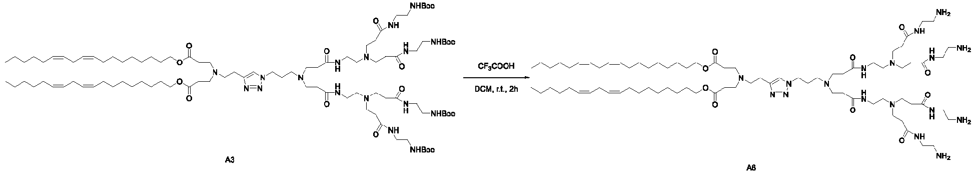

Referring to the method in Example 4, white oil A6 was synthesized from A3 as a starting material, with a yield of 69%.

MS: *m*/*z* [(M+2H⁺)/2] 748.1. ¹H NMR (500 MHz, Methanol-*d*₄) δ ppm: 7.83 (s, 1H), 5.36 (p, *J=* 7.3 Hz, 8H), 4.41 (d, *J =* 3.6 Hz, 2H), 4.07 (t, *J =* 6.7 Hz, 4H), 3.34 (d, *J =* 9.1 Hz, 8H), 3.27 (t, *J=* 6.8 Hz, 4H), 2.89 - 2.74 (m, 24H), 2.60 (t, *J=* 6.7 Hz, 4H), 2.48 (t, *J=* 6.8 Hz, 6H), 2.38 - 2.34 (m, 8H), 2.19 (dt, *J =* 24.1, 7.5 Hz, 4H), 2.08 (q, *J =* 6.2, 5.8 Hz, 12H), 1.64 (d, *J =* 7.4 Hz, 10H), 1.40 **-** 1.32 (m, 32H), 0.90 (s, 6H).

### EXAMPLE 7

This example provided dendron-like lipid compound A7. A method for preparing the dendron-like lipid compound A7 included the following steps:

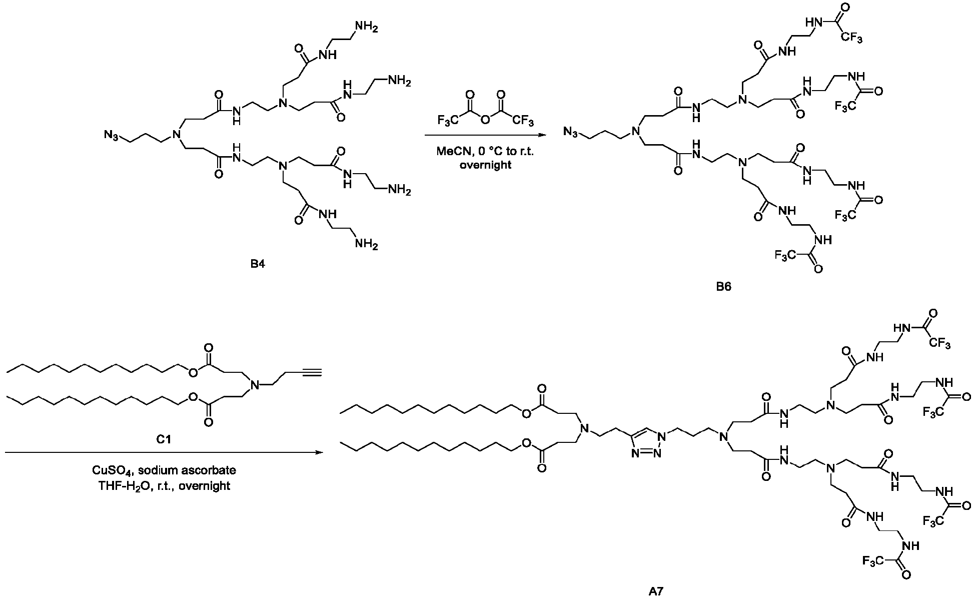

### (1) Synthesis of intermediate B6

Under argon protection, intermediate B4 (0.5 g, 0.64 mmol) was dissolved in 5 mL of anhydrous acetonitrile and stirred in an ice-water bath, and trifluoroacetic anhydride (1.4 g, 6.67 mmol) was added. The reaction was kept at a low temperature for 1 h, the ice-water bath was then removed, and the reaction continued at room temperature overnight. The resulted reaction product was concentrated under reduced pressure to dryness, and separated and purified by silica gel column [dichloromethane : methanol = 1 : 1 (V/V)] to obtain light yellow oil B6 with a yield of 50%.

MS: *m*/*z* (M+H⁺) 1169.9. ¹H NMR (500 MHz, Methanol-d4) δ ppm: 3.75 - 3.69 (m, 4H), 3.56 (dt, *J =* 12.9, 6.3 Hz, 14H), 3.49 - 3.34 (m, 26H), 2.90 (t, *J =* 6.2 Hz, 4H), 2.76 (t, *J =* 6.3 Hz, 8H), 2.12 (p, *J=* 6.7 Hz, 2H).

### (2) Synthesis of compound A7

Referring to the method in step (5) of Example 1, colorless gel A7 was synthesized from B6 and C1 as starting materials, with a yield of 48%.

MS: *m*/*z* [(M+2H⁺)/2] 859.9. ¹H NMR (500 MHz, Methanol-d4) δ ppm: 7.81 (s, 1H), 4.42 (t, *J=* 7.0 Hz, 2H), 4.07 (t, *J=* 6.6 Hz, 4H), 3.44 - 3.36 (m, 20H), 3.30 (s, 4H), 2.83 (d, *J=* 26.8 Hz, 20H), 2.61 (s, 4H), 2.49 (q, *J =* 8.5, 6.9 Hz, 6H), 2.37 (t, *J =* 6.9 Hz, 12H), 2.07 (s, 4H), 1.65 (t, *J=* 7.0 Hz, 4H), 1.31 (s, 40H), 0.92 (t, 6H).

### EXAMPLE 8

This example provided dendron-like lipid compound A8. A method for preparing the dendron-like lipid compound A8 included the following steps:

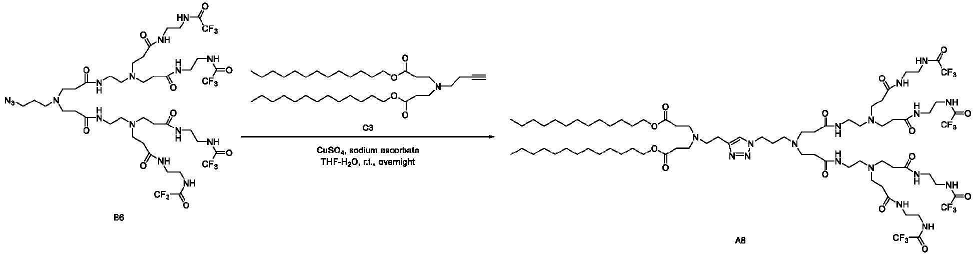

Referring to the method in step (5) of Example 1, light yellow gel A8 was synthesized from B6 and C3 as starting materials, with a yield of 44%.

MS: *m*/*z* (M+H⁺) 1746.7. ¹H NMR (500 MHz, Methanol-d4) δ ppm: 7.81 (s, 1H), 4.40 (t, *J =* 7.0 Hz, 2H), 4.05 (t, *J =* 6.7 Hz, 4H), 3.38 (dt, *J =* 21.7, 50 Hz, 20H), 3.28 (t, *J =* 6.7 Hz, 4H), 2.80 (dt, *J =* 30.2, 6.9 Hz, 20H), 2.58 (t, *J =* 6.7 Hz, 4H), 2.47 (q, *J =* 7.1, 5.4 Hz, 6H), 2.35 (q, *J =* 6.6 Hz, 12H), 2.05 (p, *J =* 6.8 Hz, 4H), 1.63 (p, *J =* 6.8 Hz, 4H), 1.41 - 1.21 (m, 44H), 0.90 (t, *J =* 6.7 Hz, 6H).

### EXAMPLE 9

This example provided dendron-like lipid compound A9. A method for preparing the dendron-like lipid compound A9 included the following steps:

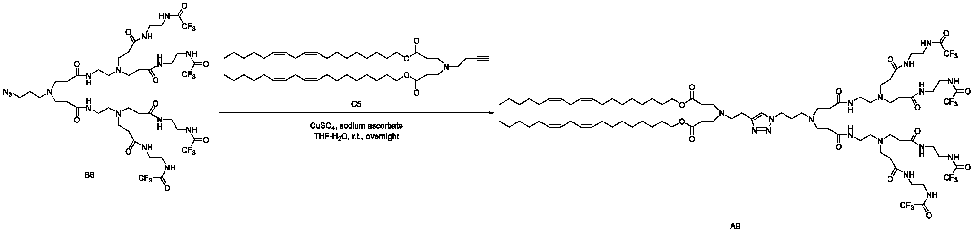

Referring to the method in step (5) of Example 1, yellow gel A9 was synthesized from B6 and C5 as starting materials, with a yield of 45%.

MS: *m*/*z* [(M+2H⁺)/2] 940.0. ¹H NMR (500 MHz, Methanol-d4) δ ppm: 7.81 (s, 1H), 5.36 (dt, *J =* 12.4, 7.6 Hz, 8H), 4.41 (s, 2H), 4.07 (t, *J =* 6.6 Hz, 4H), 3.43 - 3.35 (m, 20H), 3.29 (t, *J* = 6.4 Hz, 4H), 2.90 - 2.74 (m, 24H), 2.60 (t, *J =* 6.5 Hz, 4H), 2.48 (t, *J =* 7.1 Hz, 6H), 2.36 (t, *J* = 6.1 Hz, 12H), 2.08 (p, *J =* 7.9, 7.2 Hz, 12H), 1.65 (t, *J =* 7.1 Hz, 4H), 1.36 (dd, *J =* 24.4, 9.8 Hz, 36H), 0.94 - 0.91 (m, 6H).

### EXAMPLE 10

This example provided dendron-like lipid compound A10. A method for preparing the dendron-like lipid compound A10 included the following steps:

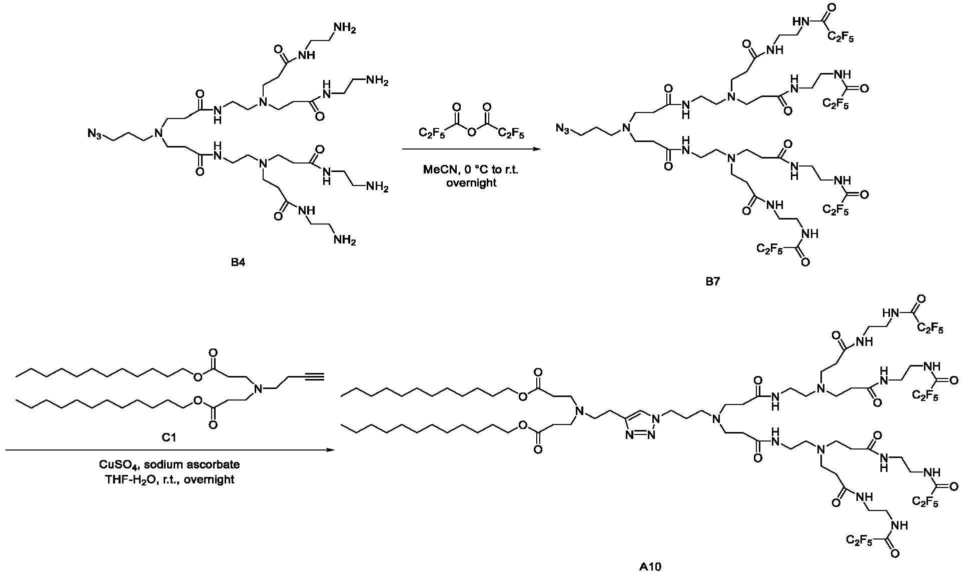

### (1) Synthesis of intermediate B7

Referring to the method in step (1) of Example 7, light yellow oil B7 was synthesized from B4 and pentafluoropropionic anhydride as starting materials, with a yield of 49%.

MS: *m*/*z* (M+H⁺) 1369.5. ¹H NMR (400 MHz, Methanol-d4) δ ppm: 3.68 (d, *J =* 5.6 Hz, 4H), 3.52 (dt, *J =* 33.3, 6.3 Hz, 20H), 3.42 - 3.34 (m, 16H), 2.89 (t, *J=* 6.2 Hz, 4H), 2.74 (t, *J* = 6.3 Hz, 8H), 2.12 (p, *J =* 6.6 Hz, 2H).

### (2) Synthesis of compound A10

Referring to the method in step (5) of Example 1, colorless gel A10 was synthesized from B7 and C1 as starting materials, with a yield of 45%.

MS: *m*/*z* [(M+2H⁺)/2] 959.6. ¹H NMR (500 MHz, Methanol-d4) δ ppm: 7.80 (s, 1H), 4.40 (t, *J =* 7.0 Hz, 2H), 4.05 (t, *J =* 6.6 Hz, 4H), 3.39 (d, *J =* 38.0 Hz, 22H), 3.28 (t, *J =* 6.6 Hz, 4H), 2.88 - 2.73 (m, 20H), 2.59 (t, *J =* 6.6 Hz, 4H), 2.47 (dt, *J =* 13.7, 6.7 Hz, 6H), 2.35 (q, *J =* 7.1 Hz, 12H), 2.05 (p, *J =* 6.7 Hz, 2H), 1.63 (p, *J =* 6.7 Hz, 4H), 1.38 - 1.26 (m, 40H), 0.90 (t, *J =* 6.8 Hz, 6H).

### EXAMPLE 11

This example provided dendron-like lipid compound A11. A method for preparing the dendron-like lipid compound A11 included the following steps:

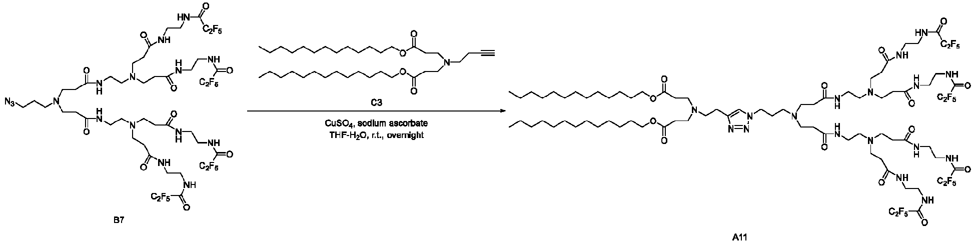

Referring to the method in step (5) of Example 1, light yellow gel A11 was synthesized from B7 and C3 as starting materials, with a yield of 41%.

MS: *m*/*z* (M+H⁺) 973.5. ¹H NMR (500 MHz, Methanol-d4) δ ppm: 7.80 (s, 1H), 4.40 (t, *J* = 7.0 Hz, 2H), 4.05 (t, *J =* 6.6 Hz, 4H), 3.39 (d, *J =* 3 8. 0 Hz, 22H), 3.28 (t, *J =* 6.6 Hz, 4H), 2.88 - 2.73 (m, 20H), 2.59 (t, *J =* 6.6 Hz, 4H), 2.47 (dt, *J =* 13.7, 6.7 Hz, 6H), 2.35 (q, *J =* 7.1 Hz, 12H), 2.05 (p, *J =* 6.7 Hz, 2H), 1.63 (p, *J=* 6.7 Hz, 4H), 1.38 -1.26 (m, 44H), 0.90 (t, *J =* 6.8 Hz, 6H).

### EXAMPLE 12

This example provided dendron-like lipid compound A12. A method for preparing the dendron-like lipid compound A12 included the following steps:

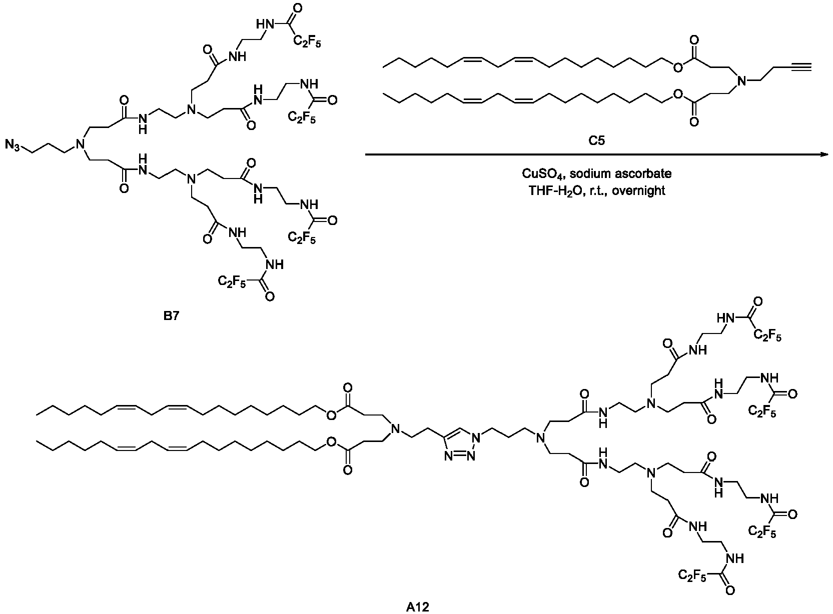

Referring to the method in step (5) of Example 1, yellow gel A12 was synthesized from B7 and C5 as starting materials, with a yield of 40%.

MS: *m*/*z* [(M+2H⁺)/2] 1039.9. ¹H NMR (500 MHz, Methanol-d4) δ ppm: 7.80 (s, 1H), 5.34 (qd, *J =* 11.2, 10.7, 4.5 Hz, 6H), 4.39 (t, *J =* 6.9 Hz, 2H), 4.05 (t, *J =* 6.6 Hz, 4H), 3.42 (t, *J=* 6.1 Hz, 8H), 3.35 (t, *J=* 6.1 Hz, 8H), 3.27 (t, *J=* 6.8 Hz, 4H), 2.86 - 2.74 (m, 22H), 2.58 (t, *J =* 6.6 Hz, 4H), 2.46 (t, *J =* 7.0 Hz, 6H), 2.35 (q, *J =* 6.6 Hz, 12H), 2.06 (q, *J =* 6.8 Hz, 8H), 1.62 (q, *J=* 6.9 Hz, 4H), 1.33 (tt, *J=* 19.3, 15.3, 5.0 Hz, 32H), 0.91 (t, *J=* 6.8 Hz, 6H).

### EXAMPLE 13

This example provided dendron-like lipid compound A13. A method for preparing the dendron-like lipid compound A13 included the following steps:

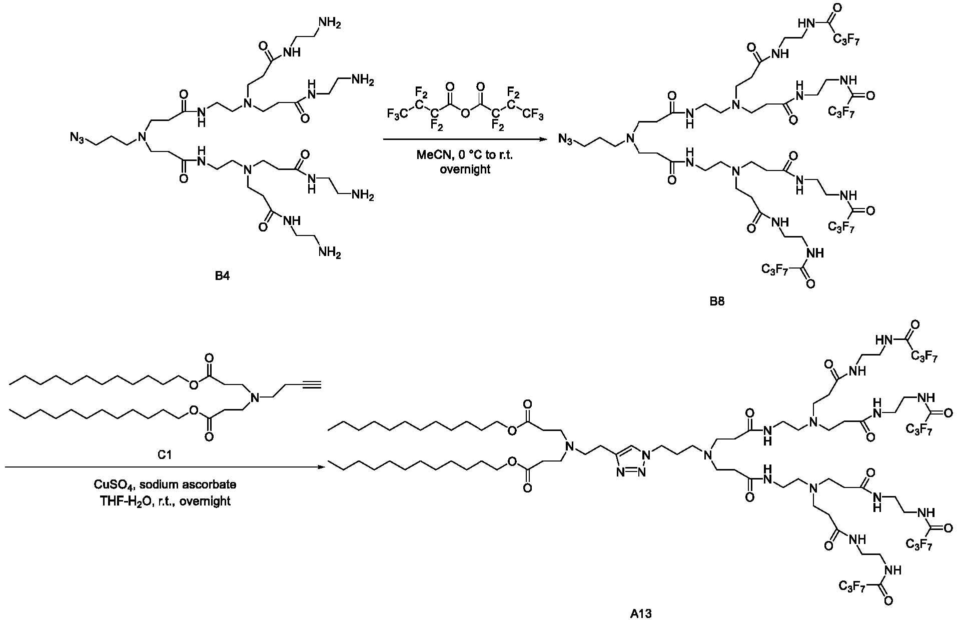

### (1) Synthesis of intermediate B8

Referring to the method in step (1) of Example 7, light yellow oil B8 was synthesized from B4 and heptafluorobutyric anhydride as starting materials, with a yield of 48%.

MS: *m*/*z* (M+H⁺) 1569.8. ¹H NMR (400 MHz, Methanol-d4) δ ppm: 3.70 (s, 4H), 3.53 (dt, *J =* 37.4, 6.3 Hz, 20H), 3.38 (d, *J =* 2.9 Hz, 16H), 2.90 (t, *J =* 6.1 Hz, 4H), 2.75 (d, *J =* 6.3 Hz, 8H), 2.14 (q, J= 7.1 Hz, 2H).

### (2) Synthesis of compound A13

Referring to the method in step (5) of Example 1, colorless gel A13 was synthesized from B8 and C1 as starting materials, with a yield of 40%.

MS: *m*/*z* [(M+2H⁺)/2] 1060.3. ¹H NMR (500 MHz, Methanol-d4) δ ppm: 7.80 (s, 1H), 4.39 (t, *J =* 7.0 Hz, 2H), 4.05 (t, *J =* 6.6 Hz, 4H), 3.39 (dt, *J =* 38.4, 6.2 Hz, 22H), 3.28 (t, *J =* 6.6 Hz, 4H), 2.87 - 2.72 (m, 20H), 2.59 (t, *J =* 6.6 Hz, 4H), 2.47 (dt, *J =* 13.7, 6.7 Hz, 6H), 2.35 (q, *J =* 6.5 Hz, 12H), 2.08 - 2.02 (m, 2H), 1.62 (q, *J =* 6.9 Hz, 4H), 1.29 (d, *J =* 4.8 Hz, 40H), 0.91 - 0.88 (m, 6H).

### EXAMPLE 14

This example provided dendron-like lipid compound A14. A method for preparing the dendron-like lipid compound A14 included the following steps:

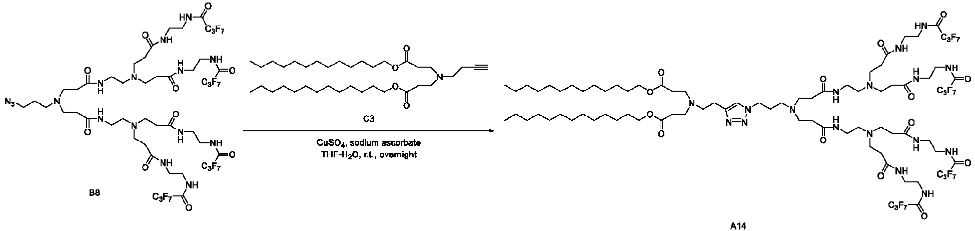

Referring to the method in step (5) of Example 1, light yellow gel A14 was synthesized from B8 and C3 as starting materials, with a yield of 47%.

MS: *m*/*z* (M+H⁺) 1075.0. ¹H NMR (500 MHz, Methanol-d4) δ ppm: 7.80 (s, 1H), 4.39 (t, *J =* 7.0 Hz, 2H), 4.05 (t, *J =* 6.6 Hz, 4H), 3.39 (dt, *J =* 38.4, 6.2 Hz, 22H), 3.28 (t, *J =* 6.6 Hz, 4H), 2.87 - 2.72 (m, 20H), 2.59 (t, *J=* 6.6 Hz, 4H), 2.47 (dt, *J =* 13.7, 6.7 Hz, 6H), 2.35 (q, *J=* 6.5 Hz, 12H), 2.08 - 2.02 (m, 2H), 1.62 (q, *J =* 6.9 Hz, 4H), 1.32 (d, *J =* 4.8 Hz, 44H), 0.92 - 0.87(m, 6H).

### EXAMPLE 15

This example provided dendron-like lipid compound A15. A method for preparing the dendron-like lipid compound A15 included the following steps:

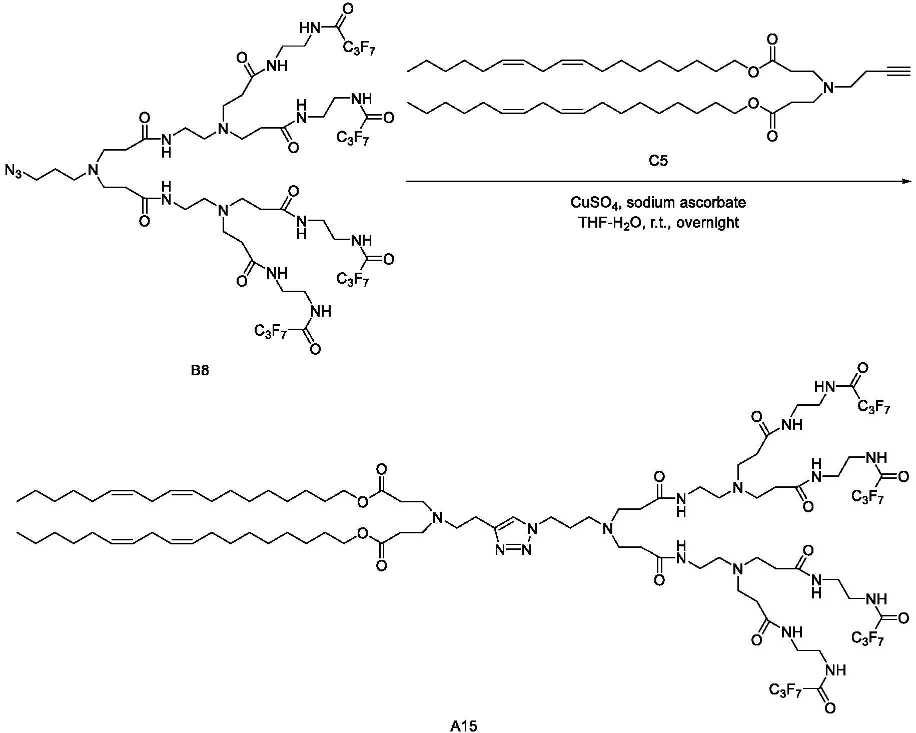

Referring to the method in step (5) of Example 1, yellow gel A15 was synthesized from B8 and C5 as starting materials, with a yield of 38%.

MS: *m*/*z* [(M+2H⁺)/2] 1140.4. ¹H NMR (500 MHz, Methanol-d4) δ ppm: 7.80 (s, 1H), 5.40 - 5.28 (m, 8H), 4.39 (t, *J=* 7.0 Hz, 2H), 4.05 (t, *J =* 6.6 Hz, 4H), 3.39 (dt, *J=* 38.7, 6.3 Hz, 22H), 3.27 (d, *J =* 6.5 Hz, 4H), 2.85 - 2.74 (m, 20H), 2.60 (t, *J =* 6.7 Hz, 4H), 2.47 (q, *J =* 8.7, 6.9 Hz, 6H), 2.36 (q, *J =* 6.9 Hz, 12H), 2.06 (q, *J =* 7.0 Hz, 10H), 1.62 (q, *J* = 7.0 Hz, 4H), 1.38 - 1.29 (m, 40H), 0.90 (d, *J =* 7.1 Hz, 6H).

### EXAMPLE 16

This example provided dendron-like lipid compound A16. A method for preparing the dendron-like lipid compound A16 included the following steps:

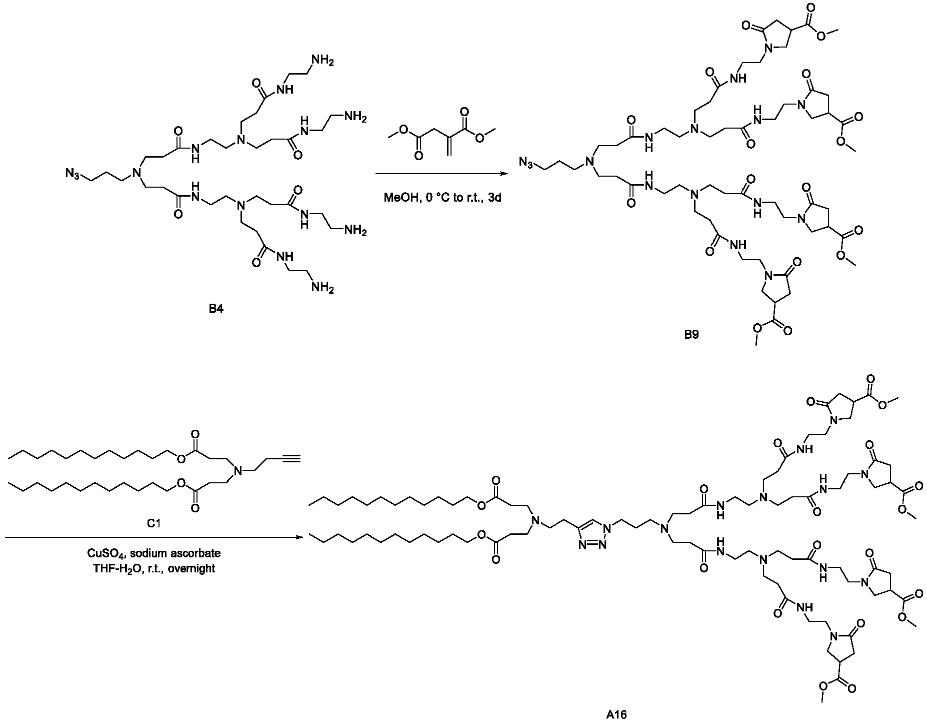

### (1) Synthesis of intermediate B9

Under argon protection, intermediate B4 (1.0 g, 1.27 mmol) was dissolved in 9 mL of anhydrous methanol and stirred in an ice-water bath, and 6 mL of anhydrous methanol dissolved with dimethyl itaconate (0.96 g, 6.07 mmol) was slowly added dropwise. After the addition was completed, the reaction was kept at a low temperature for 1 h, the ice-water bath was then removed, and the reaction was continued at room temperature for 3 days. After concentration under reduced pressure to dryness, 10 mL of diethyl ether and 10 mL of water were added and fully stirred, and the aqueous phase was taken and freeze-dried to obtain light yellow oil B9 with a yield of 40%.

MS: *m*/*z* (M+H⁺) 1289.8. ¹H NMR (400 MHz, Methanol-d4) δ ppm: 3.84 - 3.75 (m, 18H), 3.71 (q, *J =* 5.7 Hz, 6H), 3.40 (d, *J =* 18.4 Hz, 30H), 3.31 (t, *J =* 6.5 Hz, 4H), 2.80 (t, *J =* 6.8 Hz, 12H), 2.71 - 2.54 (m, 14H), 2.38 (dt, *J =* 18.2, 6.8 Hz, 12H).

### (2) Synthesis of compound A16

Referring to the method in step (5) of Example 1, colorless gel A16 was synthesized from B9 and C1 as starting materials, with a yield of 47%.

MS: *m*/*z* [(M+2H⁺)/2] 920.4. ¹H NMR (500 MHz, Methanol-d4) δ ppm: 7.84 (s, 1H), 4.42 (t, *J =* 7.1 Hz, 2H), 4.07 (t, *J =* 6.7 Hz, 4H), 3.82 - 3.76 (m, 4H), 3.75 (s, 12H), 3.69 (dd, *J =* 9.9, 5.8 Hz, 4H), 3.47 - 3.35 (m, 22H), 3.30 (d, *J =* 6.5 Hz, 4H), 2.82 (dt, *J =* 32.5, 6.6 Hz, 20H), 2.69 - 2.58 (m, 12H), 2.50 (dt, *J =* 13.8, 6.9 Hz, 6H), 2.37 (dt, *J =* 22.6, 6.9 Hz, 12H), 2.07 (p, *J =* 7.0 Hz, 2H), 1.65 (p, *J =* 6.7 Hz, 4H), 1.41 - 1.29 (m, 40H), 0.92 (t, *J =* 6.9 Hz, 6H).

### EXAMPLE 17

This example provided dendron-like lipid compound A17. A method for preparing the dendron-like lipid compound A17 included the following steps:

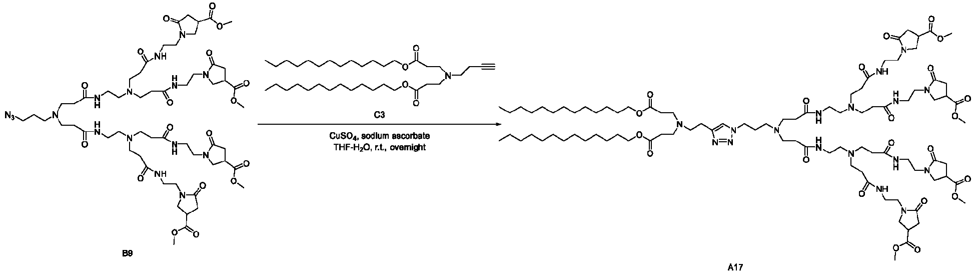

Referring to the method in step (5) of Example 1, yellow gel A17 was synthesized from B9 and C3 as starting materials, with a yield of 40%.

MS: *m*/*z* (M/2+Na⁺) 956.2. ¹H NMR (500 MHz, Methanol-d4) δ ppm: 7.84(s, 1H), 4.42 (t, *J =* 7.1 Hz, 2H), 4.07 (t, *J =* 6.7 Hz, 4H), 3.82 - 3.76 (m, 4H), 3.75 (s, 12H), 3.69 (dd, *J=* 9.9, 5.8 Hz, 4H), 3.47 - 3.35 (m, 22H), 3.30 (d, *J =* 6.5 Hz, 4H), 2.82 (dt, *J =* 32.5, 6.6 Hz, 20H), 2.69 - 2.58 (m, 12H), 2.50 (dt, *J =* 13.8, 6.9 Hz, 6H), 2.37 (dt, *J =* 22.6, 6.9 Hz, 12H), 2.07 (p, *J=* 7.0 Hz, 2H), 1.65 (p, *J=* 6.7 Hz, 4H), 1.41 - 1.29 (m, 44H), 0.92 (t, *J=* 6.9 Hz, 6H).

### EXAMPLE 18

This example provided dendron-like lipid compound A18. A method for preparing the dendron-like lipid compound A18 included the following steps:

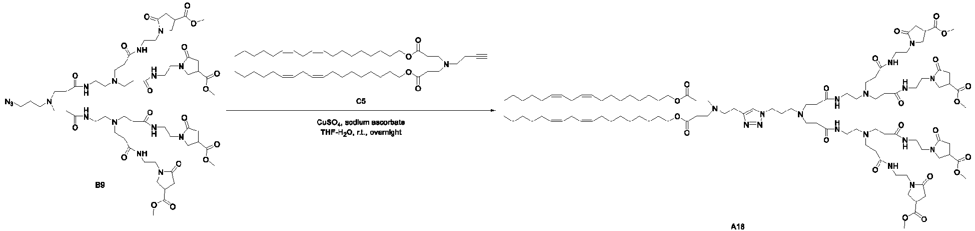

Referring to the method in step (5) of Example 1, yellow gel A18 was synthesized from B9 and C5 as starting materials, with a yield of 35%.

MS: *m*/*z* [(M+2H⁺)/2] 1000.8. ¹H NMR (500 MHz, Methanol-d4) δ ppm: 7.83 (s, 1H), 5.42 - 5.30 (m, 8H), 4.41 (t, *J =* 7.0 Hz, 2H), 4.08 (t, *J =* 6.6 Hz, 4H), 3.79 (d, *J =* 9.7 Hz, 4H), 3.75 (s, 12H), 3.69 (dd, *J =* 9.9, 5.8 Hz, 4H), 3.48 - 3.34 (m, 20H), 3.30 (t, *J =* 6.5 Hz, 4H), 2.82 (dt, *J =* 36.4, 6.8 Hz, 24H), 2.70 - 2.63 (m, 8H), 2.60 (t, *J =* 6.7 Hz, 4H), 2.49 (dt, *J =* 13.5, 6.8 Hz, 6H), 2.36 (dt, *J =* 20.9, 6.8 Hz, 12H), 2.08 (p, *J =* 6.8 Hz, 10H), 1.65 (p, *J =* 6.7 Hz, 4H), 1.42 - 1.32 (m, 32H), 0.93 (t, *J =* 6.8 Hz, 6H).

### EXAMPLE 19

This example provided dendron-like lipid compound A19. A method for preparing the dendron-like lipid compound A19 included the following steps:

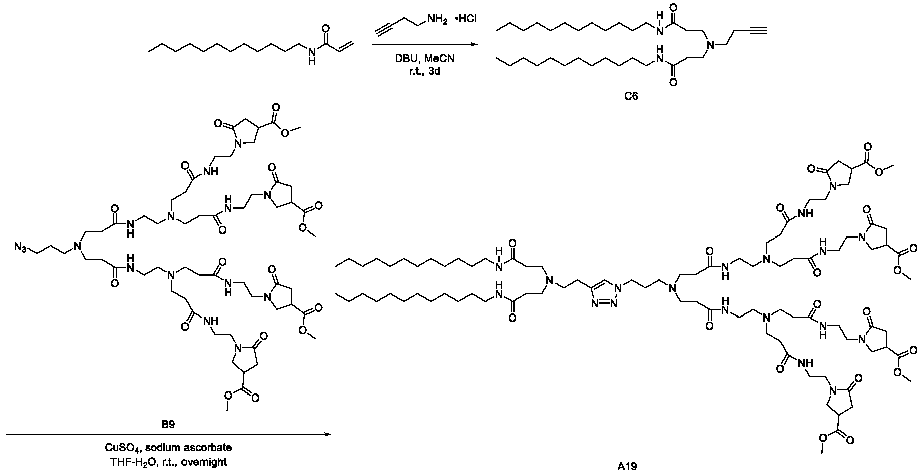

### (1) Synthesis of intermediate C6

Referring to the method in step (5) of Example 1, yellow oil C6 was synthesized from N-dodecylacrylamide as a starting material, with a yield of 30%.

MS: *m*/*z* (M+H⁺) 548.9. ¹H NMR (400 MHz, Chloroform-*d*) δ ppm: 3.17 (t, *J =* 7.4 Hz, 4H), 2.81 (t, *J =* 6.7Hz, 6H), 2.77 (s, 1H), 2.43 (t, *J =* 6.7 Hz, 6H), 1.56 (tt, *J =* 7.4, 7.0 Hz, 4H), 1.36-1.17 (m, 36H), 0.87 (t, *J =* 7.0 Hz, 6H).

### (2) Synthesis of compound A19

Referring to the method in step (5) of Example 1, colorless gel A19 was synthesized from B9 and C6 as starting materials, with a yield of 45%.

MS: *m*/*z* (M/2+Na⁺) 941.2. ¹H NMR (500 MHz, Methanol-d4) δ ppm: 7.86 (s, 1H), 4.43 (t, *J =* 7.1 Hz, 2H), 3.78 (d, *J* = 15.6 Hz, 18H), 3.71 (d, *J* = 3.6 Hz, 4H), 3.46 - 3.39 (m, 18H), 3.31 (d, *J =* 6.5 Hz, 4H), 3.18 (t, *J =* 7.2 Hz, 4H), 2.89 - 2.77 (m, 20H), 2.74 - 2.64 (m, 10H), 2.62 (t, *J =* 6.9 Hz, 4H), 2.52 (t, *J =* 6.8 Hz, 2H), 2.38 (dd, *J =* 15.6, 6.7 Hz, 14H), 2.14 - 2.02 (m, 2H), 1.52 (t, *J =* 7.0 Hz, 4H), 1.34 (d, *J =* 15.3 Hz, 36H), 0.93 (t, *J =* 6.8 Hz, 6H).

### EXAMPLE 20

This example provided dendron-like lipid compound A20. A method for preparing the dendron-like lipid compound A20 included the following steps:

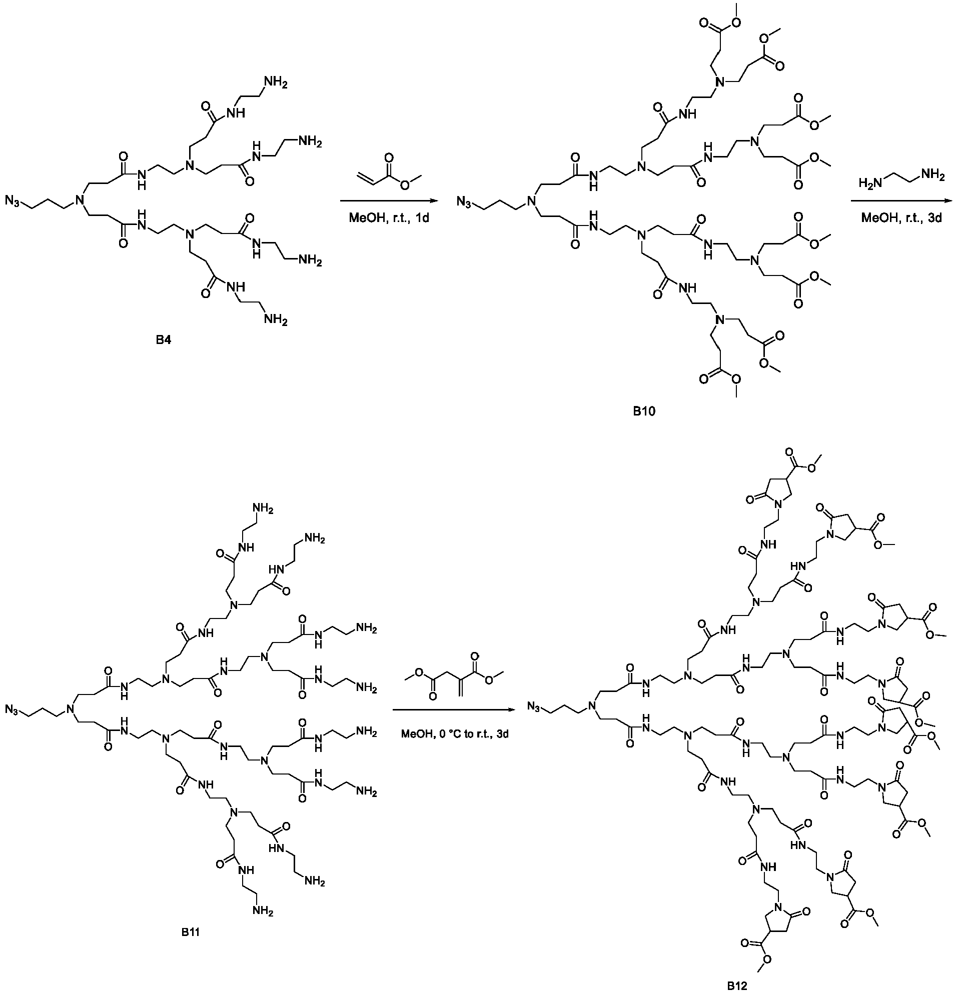

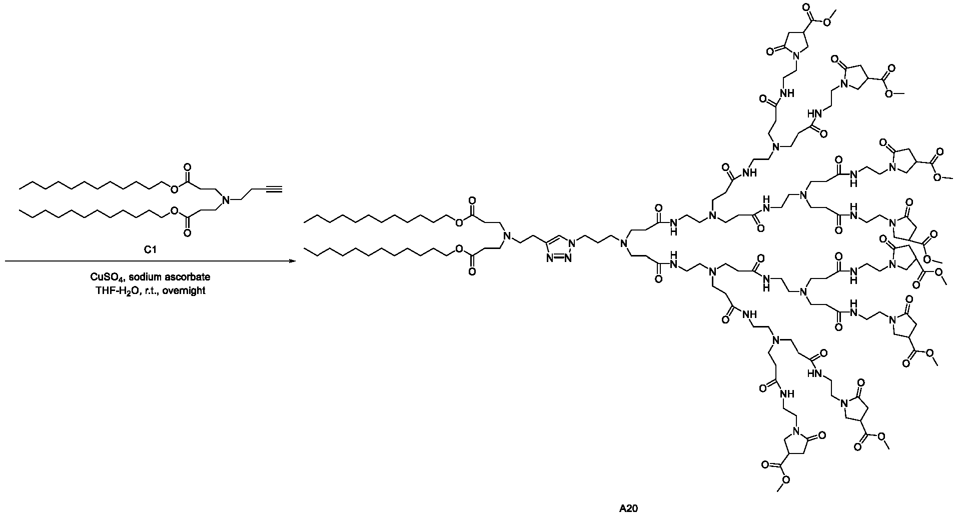

### (1) Synthesis of intermediate B12

Referring to the methods in Examples 1 and 16, B4 as a starting material was reacted with methyl acrylate to obtain intermediate B10, which was in turn reacted with ethylenediamine to obtain intermediate B11, which was finally reacted with dimethyl itaconate to obtain yellow oil B12.

MS: *m*/*z* (M/2+Na⁺) 1376.7.

### (2) Synthesis of compound A20

Referring to the method in step (5) of Example 1, yellow oil A20 was synthesized from B12 and C1 as starting materials, with a yield of 39%.

MS: *m*/*z* [(M+2H⁺)/2] 1628.4633. ¹H NMR (500 MHz, Methanol-d4) δ ppm: 7.84 (s, 1H), 4.43 (d, *J =* 7.1 Hz, 2H), 4.10 - 4.05 (m, 4H), 3.79 (t, *J =* 9.5 Hz, 16H), 3.76 (m, 20H), 3.70 (dd, *J =* 9.7, 6.3 Hz, 16H), 3.41 (dq, *J =* 18.1, 9.9, 9.5 Hz, 40H), 3.30 (d, *J =* 6.9 Hz, 16H), 2.90 - 2.77 (m, 18H), 2.64 (dd, *J =* 25.2, 7.8 Hz, 34H), 2.54 (s, 4H), 2.50 (d, *J =* 6.8 Hz, 6H), 2.39 (dt, *J =* 25.7, 7.5 Hz, 16H), 2.24 (s, 2H), 2.11 - 2.06 (m, 2H), 1.77 (q, *J =* 7.3 Hz, 2H), 1.65 (t, *J* = 7.2 Hz, 4H), 1.32 (d, *J =* 7.7 Hz, 36H), 0.95 - 0.90 (m, 6H).

### EXAMPLE 21

This example provided dendron-like lipid compound A21. A method for preparing the dendron-like lipid compound A21 included the following steps:

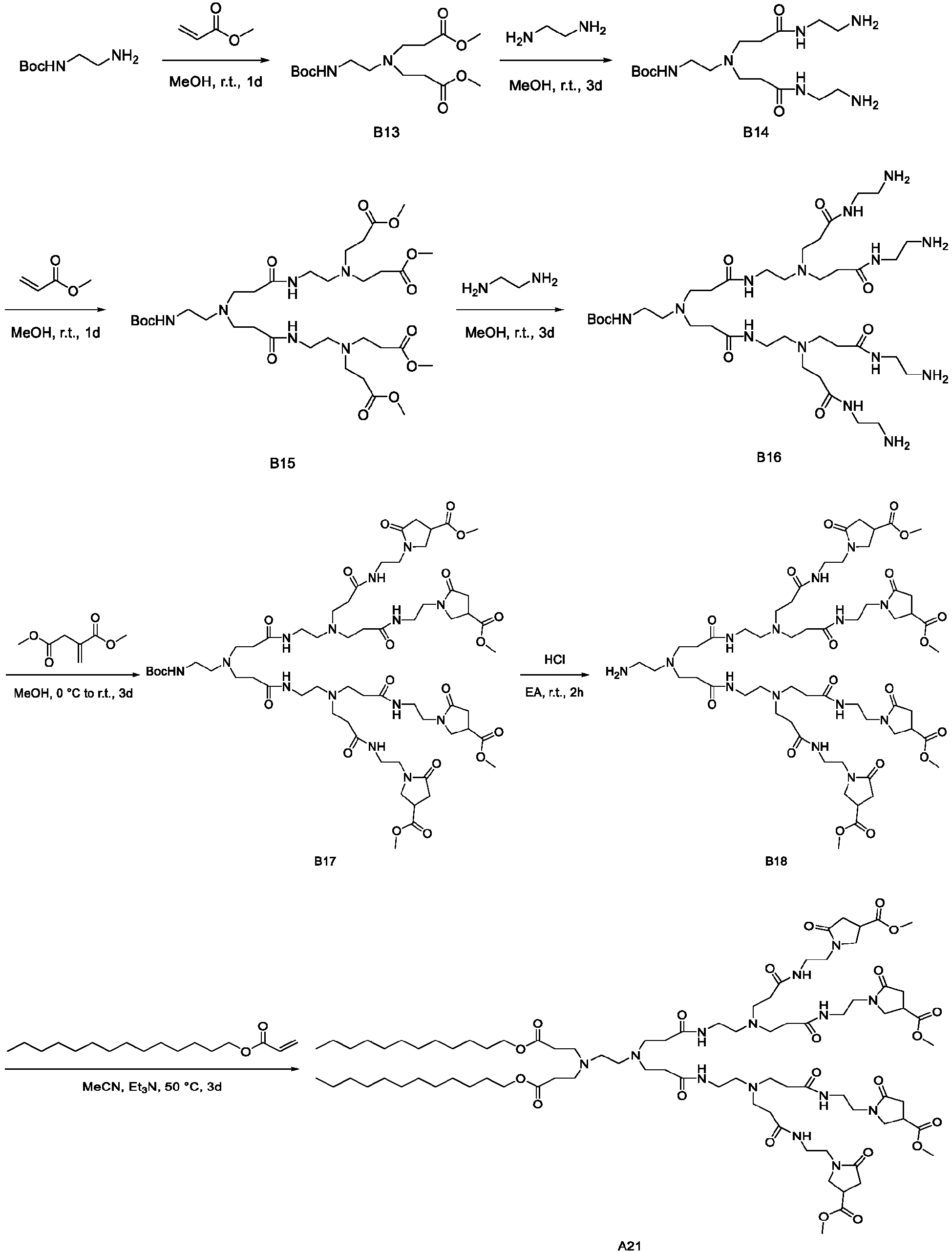

### (1) Synthesis of intermediate B 13

Referring to the method in step (1) of Example 1, colorless oil B 13 was synthesized from mono-Boc ethylenediamine as a starting material, with a yield of 94%.

MS: *m*/*z* (M+H⁺) 333.4. ¹H NMR (500 MHz, Chloroform-*d*) δ ppm: 3.66 (s, 6H), 3.15 (q, *J =* 5.7 Hz, 2H), 2.72 (t, *J =* 6.8 Hz, 4H), 2.50 (t, *J =* 5.6 Hz, 2H), 2.41 (t, *J =* 6.7 Hz, 4H), 1.42 (s, 9H).

### (2) Synthesis of intermediate B15

Referring to the method in step (2) of Example 1, light yellow oil B15 was synthesized from B13 as a starting material, with a yield of 90%.

MS: *m*/*z* (M+H⁺) 733.5. ¹H NMR (500 MHz, Chloroform-*d*) δ ppm: 3.69 (s, 12H), 3.29 (t, *J =* 6.3 Hz, 4H), 3.16 (t, *J =* 6.5 Hz, 2H), 2.81 (dt, *J =* 13.3, 6.8 Hz, 12H), 2.59 (td, *J =* 6.3, 3.3 Hz, 6H), 2.49 (t, *J* = 6.7 Hz, 8H), 2.39 (t, *J =* 6.9 Hz, 4H), 1.45 (s, 9H).

### (3) Synthesis of intermediate B17

Referring to the methods in Example 1 and Example 16, B15 as a starting material was reacted with ethylenediamine to obtain intermediate B16, which was then reacted with dimethyl itaconate to obtain yellow oil B17.

MS: *m*/*z* (M+H⁺) 1531.0. ¹H NMR (400 MHz, Methanol-d4) δ 3.81 (d, *J =* 9.5 Hz, 3H), 3.77 (s, 12H), 3.71 (dd, *J* = 10.0, 5.8 Hz, 4H), 3.47 - 3.36 (m, 24H), 3.31 (d, *J =* 6.5 Hz, 3H), 3.17 (t, *J =* 6.6 Hz, 2H), 2.83 (dt, *J =* 13.7, 6.9 Hz, 12H), 2.72 - 2.58 (m, 14H), 2.39 (dt, *J*= 17.5, 6.9 Hz, 12H), 1.47 (s, 9H).

### (4) Synthesis of intermediate B18

Under argon protection, B17 (50 mg, 0.037 mmol) was dissolved in 2 mL of a 4M hydrogen chloride ethyl acetate solution with stirring in an ice water bath and then gradually returned to room temperature for reaction overnight. After concentration under reduced pressure to dryness, yellow-white solid B18 hydrochloride was obtained with a yield of 88%.

MS: *m*/*z* [(M+2H⁺)/2] 625.9. ¹H NMR (400 MHz, Methanol-d4) δ 3.74 (d, *J =* 28.9 Hz, 32H), 3.58 (s, 10H), 3.43 (dd, *J =* 23.3, 9.8 Hz, 24H), 2.99 (d, *J =* 6.0 Hz, 4H), 2.77 (dd, *J* = 29.1, 8.3 Hz, 14H).

### (5) Synthesis of compound A21

Under argon protection, B18 (42 mg, 0.033 mmol) was dissolved in 2 mL of anhydrous acetonitrile, and triethylamine (20 µL, 0.143 mmol) was added and stirred at room temperature for 0.5 h. Subsequently, tetradecyl acrylate (100 µL, 0.324 mmol) was added, and the temperature was raised to 50°C for reaction for 3 d. The resulted reaction product was concentrated under reduced pressure to dryness, and separated and purified by silica gel column [dichloromethane : methanol = 10 : 1 (V/V)] to obtain light yellow oil A21 with a yield of 30%.

MS: *m*/*z* [(M+2H⁺)/2] 893.6.

### APPLICATION EXAMPLE 1

### Preparation of liposomes

The synthesized dendron-like lipid compounds (A7-A18), DSPC, cholesterol and DMG-PEG2000 were respectively dissolved in anhydrous ethanol for later use. The above solutions were mixed at a molar ratio of 50 : 10 : 35 : 5. Under stirring, the alcohol phase solution prepared above was dropwise added to PBS, with the volume ratio of alcohol phase to PBS being 1 : 3, and after mixing, the materials were stirred for 1 minute. The reaction solution was added to a 10 K MVCO ultrafiltration centrifuge tube, PBS was added, and the tube was balanced. A high-speed centrifuge was used for centrifugation at 4000 g for 25 minutes. After the solution was concentrated, PBS was added again for centrifugation at 4000 g for 25 minutes, and solution displacement was carried out to finally obtain a liposome solution.

The particle size and surface potential of the liposome was characterized by detection with Malvern particle size analyzer. The liposome solution was prepared into a 200 µg/mL aqueous solution with PBS. In addition, 16:0 NBD PE containing 1% fluorescent molecules was added to the above liposome as a model drug to prepare a liposome with a fluorescence label. The particle size and potential of the obtained liposome were measured by Malvern particle size analyzer. The test results were as shown in Table 1:

**Table 1**

| Dendron-like lipid compound in liposome | Particle size (nm) | Polydispersity | Surface charge (mV) |
|---|---|---|---|
| A7 | 95.75±0.989 | 0.272 | -5.56±1.675 |
| A8 | 114.17±0.115 | 0.203 | -2.10±1.240 |
| A9 | 88.53±0.689 | 0.168 | -4.91±0.936 |
| A10 | 93.73±0.423 | 0.102 | -7.11±0.972 |
| A11 | 123.37±0.808 | 0.103 | -7.96±3.195 |
| A12 | 99.58±0.285 | 0.096 | -7.19±2.274 |
| A13 | 162.67±1.159 | 0.107 | -7.19±0.979 |
| A14 | 150.83±2.316 | 0.113 | -7.60±4.34 |
| A15 | 173.10±2.553 | 0.118 | -7.39±2.033 |
| A16 | 77.92±1.202 | 0.142 | -10.71±3.42 |
| A17 | 69.39±0.238 | 0.165 | -12.85±4.249 |
| A18 | 85.33±3.699 | 0.241 | -8.48±0.360 |

As shown in Table 1, the size of the synthesized liposome was centered between 69 nm and 170 nm, and the polydispersity index (PDI) was basically less than 0.3, indicating that the prepared liposome particles had relatively good uniformity. Among them, the PYR dendron-like head-related liposomes had the smallest particle size, whereas the 7F-related liposomes had the largest particle size. For example, A16 had a particle size of 77.92±1.202 nm and A13 had a particle size of 162.67±1.159 nm. All the liposomes carried negative surface charges.

### APPLICATION EXAMPLE 2

### Preparation of siRNA-encapsulating liposomes

The synthesized dendron-like lipid compounds (A1, A4, A8, A10, A18, and A19), DSPC, cholesterol and DMG-PEG2000 were respectively dissolved in anhydrous ethanol for later use. The above solutions were mixed at a molar ratio of 50 : 10 : 38.5 : 1.5. siRNA was dissolved in a sodium citrate solution with pH = 4.0. Under stirring, the alcohol phase solution prepared above was dropwise added to an siRNA-sodium citrate solution, with the volume ratio of alcohol phase to aqueous phase being 1 : 3, and after mixing, the materials were stirred for 1 minute. The reaction solution was added to a 10 K MVCO ultrafiltration centrifuge tube, PBS was added, and the tube was balanced. A high-speed centrifuge was used for centrifugation at 4000 g for 25 minutes. After the solution was concentrated, PBS was added again for centrifugation at 4000 g for 25 minutes, and solution displacement was carried out to finally obtain a liposome solution. The particle size and surface potential of the liposome was characterized by detection with Malvern particle size analyzer. The test results were as shown in Table 2:

**Table 2**

| Preparation of dendron-like lipid compound in siRNA-encapsulating liposome | Particle size (nm) | Polydispersity | Surface charge (mV) |
|---|---|---|---|
| A1 | 174.4±2.22 | 0.162 | -7.95±0.89 |
| A4 | 211.2±8.22 | 0.359 | -17.6±1.99 |
| A8 | 88.43±1.29 | 0.103 | -19.83±0.38 |
| A10 | 158.3±0.47 | 0.084 | -11.7±1.18 |
| A18 | 87.47±0.60 | 0.097 | -17.67±0.85 |
| A19 | 89.23±2.26 | 0.141 | -20.83±0.90 |

As shown in Table 2, the size of the synthesized liposome was centered between 87 nm and 211 nm, and the polydispersity index (PDI) was basically less than 0.2, indicating that the prepared siRNA-encapsulating liposome particles had relatively good uniformity. All the liposomes carried negative charges on the surface.

### APPLICATION EXAMPLE 3

### Preparation of mRNA-encapsulating liposomes

The synthesized dendron-like lipid compounds (A8, A10, and A12), DSPC, cholesterol and DMG-PEG2000 were respectively dissolved in anhydrous ethanol for later use. The above solutions were mixed at a molar ratio of 50 : 10 : 38.5 : 1.5. mRNA was dissolved in a sodium acetate solution with pH = 5.0. Under stirring, the alcohol phase solution prepared above was dropwise added to an mRNA-sodium acetate solution, with the volume ratio of alcohol phase to aqueous phase being 1 : 3, and after mixing, the materials were stirred for 1 minute. The reaction solution was added to a 10 K MVCO ultrafiltration centrifuge tube, PBS was added, and the tube was balanced. A high-speed centrifuge was used for centrifugation at 4000 g for 25 minutes. After the solution was concentrated, PBS was added again for centrifugation at 4000 g for 25 minutes, and solution displacement was carried out to finally obtain a liposome solution. The particle size and surface potential of the liposome was characterized by detection with Malvern particle size analyzer. The test results were as shown in Table 3:

**Table 3**

| Dendron-like lipid compound in mRNA-encapsulating liposome | Particle size (nm) | Polydispersity | Surface charge (mV) |
|---|---|---|---|
| A8 | 260.03±9.682 | 0.179±0.008 | -6.21±0.614 |
| A10 | 208.7±24.857 | 0.240±0.041 | -16.90±1.67 |
| A12 | 289.8±0.557 | 0.300±0.021 | -9.87±1.834 |

As shown in Table 3, the size of the synthesized liposome was centered between 208 nm and 289 nm, and the polydispersity index (PDI) was basically less than 0.3, indicating that the prepared mRNA-encapsulating liposome particles had relatively good uniformity. All the liposomes carried negative charges on the surface.

### APPLICATION EXAMPLE 4

### Evaluation of cellular uptake of liposomes

Fluorescence microscope: RAW264.7 cells were plated on a 24-well plate with the cell number being 5 × 10⁴ cells/well and cultured at 37°C under the conditions of 95% relative humidity and 5% CO₂. After 24 hours of culturing, 100 µL of a solution of a fluorescent liposome (i.e., fluorescent lipid nanoparticles) at a concentration of 1 mg/mL was added, 900 µL of a medium was then added, and incubation was carried out at 37°C under the conditions of 95% relative humidity and 5% CO₂. After 24 hours of incubation, the liquid was sucked out, and it was washed twice with PBS. 500 µL of 1X hoechst 33342 living cell dye solution was added for dyeing in the dark for 10 minutes, it was then washed with PBS 2-3 times, 1 mL of the medium was added, and it was observed under a microscope. 16:0 NBD PE could be excited at 488 nm, and Hoechst could be excited at 346 nm. It was observed with a 40X microscope. The number of A549 cells plated was 4 × 10⁴ cells/well, and the rest were consistent with RAW264.7. Among them, Hoechst, a fluorescent dye that could strongly bind to DNA in living cells, was used to label living cells. LNP were lipid nanoparticles, and Merge was superposition.

Flow cytometry: RAW264.7 cells were plated on a 24-well plate, with the cell number being 5 × 10⁵ cells/well and 3 replication wells being set. Culturing was carried out at 37°C under the conditions of 95% relative humidity and 5% CO₂. After 24 hours of culturing, 100 µL of a solution of a fluorescent liposome (i.e., fluorescent lipid nanoparticles) at a concentration of 1 mg/ml was added, 900 µL of a medium was then added, and incubation was carried out at 37°C under the conditions of 95% relative humidity and 5% CO₂. After 24 hours of incubation, the liquid was sucked out, it was washed with PBS 3 times, and the cells were blown down, collected in a 1.5 mL Ep tube, and centrifuged at 200 g for 6 minutes. After discarding the supernatant, PBS was added again to resuspend the cells, the mixture was centrifuged at 200 g again for 6 minutes, the supernatant was discarded, 300 µL of PBS was added to resuspend the cells, and filtration was carried out with a 70 µm cell filter to prepare a single cell suspension. The number of A549 cells plated was 2 × 10⁵ cells/well, and they were cultured and incubated analogously to RAW264.7 cells. After 24 hours of incubation, the liquid was sucked out, it was washed with PBS 3 times, and the cells were digested with pancreatin, collected in a 1.5 mL Ep tube, and centrifuged at 200 g for 6 minutes. After discarding the supernatant, PBS was added again to resuspend the cells, the mixture was centrifuged at 200 g again for 6 minutes, the supernatant was discarded, 300 µL of PBS was added to resuspend the cells, and filtration was carried out with a 70 µm cell filter to prepare a single cell suspension. The fluorescence intensity of the cells was analyzed by flow cytometer. The final result was calculated using relative fluorescence intensity: relative fluorescence intensity = (fluorescence intensity in experimental group - fluorescence intensity in control group) / fluorescence intensity in control group.

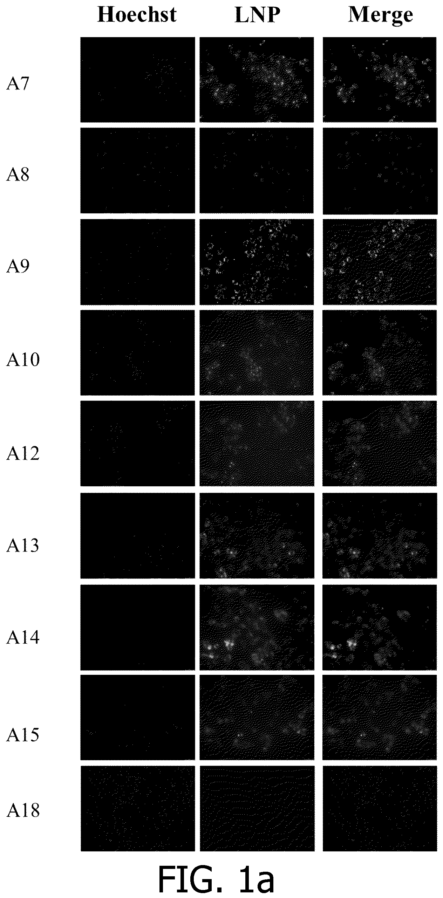

As shown in FIG. 1a, macrophages were photographed by a fluorescence microscope after being treated with different liposomes for 24 h, and the uptake could be reflected by fluorescence intensity. It could be seen that PYR group and CH₃ were hardly absorbed by macrophages. A large number of F-bearing groups were ingested by macrophages, and obvious green fluorescence could be observed in cytoplasm. Among them, A9 and A14 had the most significant effects.

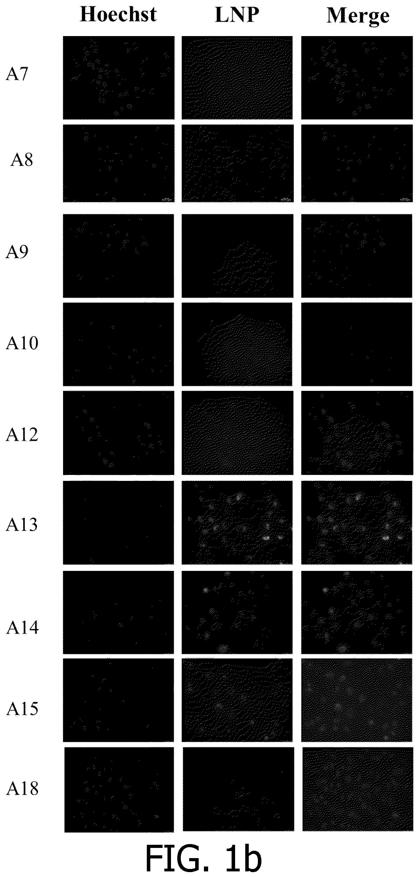

As shown in FIG. 1b, A549 cancer cells were photographed by fluorescence microscope after being treated with different liposomes for 24 h. As could be seen from the figure, most liposomes barely entered cancer cells, and the PYR surface group dendron-like head nanoliposome particles were also not ingested by cancer cells. 3F and 5F liposomes with different carbon chain lengths are not ingested by cancer cells, whereas 7F liposomes could enter cancer cells, and A13, which was the strongest, was also negatively correlated with the carbon chain length.

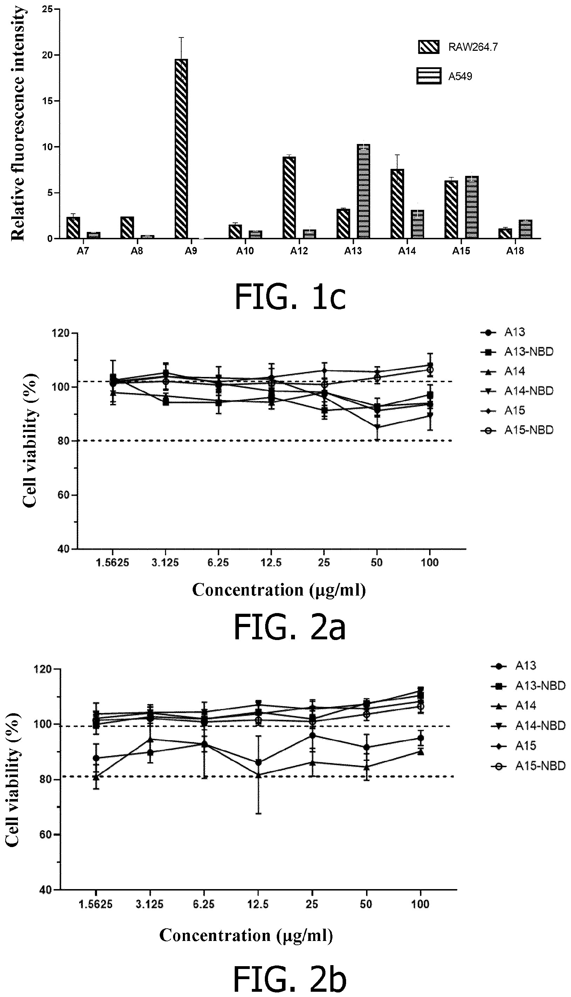

As shown in FIG. 1c, there was a difference in the uptake of liposomes by cancer cells and macrophages.

### APPLICATION EXAMPLE 5

### Evaluation of cytotoxicity of liposomes

In order to assess the cytotoxicity and cellular uptake of different liposomes, we selected two cells, RAW264.7 cells (mouse monocyte macrophage leukemia cells) and A549 cells (human lung adenocarcinoma cells), for study.

After being resuscitated, the cells were cultured at 37°C under the conditions of 95% relative humidity and 5% CO₂. RAW264.7 cells were cultured in DMEM high glucose medium + 10% FBS. A549 cells were cultured in F-12K medium + 10% FBS. Liposome PBS solutions with 7 concentrations, i.e., 1 mg/mL, 500 µg/mL, 250 µg/mL, 125 µg/mL, 62.5 µg/mL, 31.25 µg/mL, and 15.625 µg/mL, respectively, were prepared. The cells were plated in a 96-well plate, and totally 9 groups, including a blank group, a control group and 7 experimental group, were set up. Among them, the blank group was not inoculated with cells, and the control group and the experimental groups were all inoculated with 5 × 10³ cells/well, with 100 µL per well, with 6 replication wells being set for each group. They were cultured for 24 h at 37°C under the conditions of 95% relative humidity and 5% CO₂. After 24 hours of culturing, the medium for the cells were replaced, and 90 µL of a medium was added to all the groups. For the blank group and the control group, 10 µL of a PBS solution was added, and for the experimental groups, 10 µL of the liposome PBS solution was added. Culturing was continued at 37°C under the conditions of 95% relative humidity and 5% CO₂. After 24 h, CCK8 method was used for detection, wherein 10 µL of a CCK8 solution was added to each well, and it was left to stand at 37°C for 1-2 h. The absorption value was detected by enzyme-linked immunosorbent assay at 450 nm. After removing the maximum and minimum values from each group, the remaining four values were calculated according to cell survival rate (%) = [(experimental group - blank group) / (control group - blank group)] × 100% to assess the cytotoxicity of the liposomes.

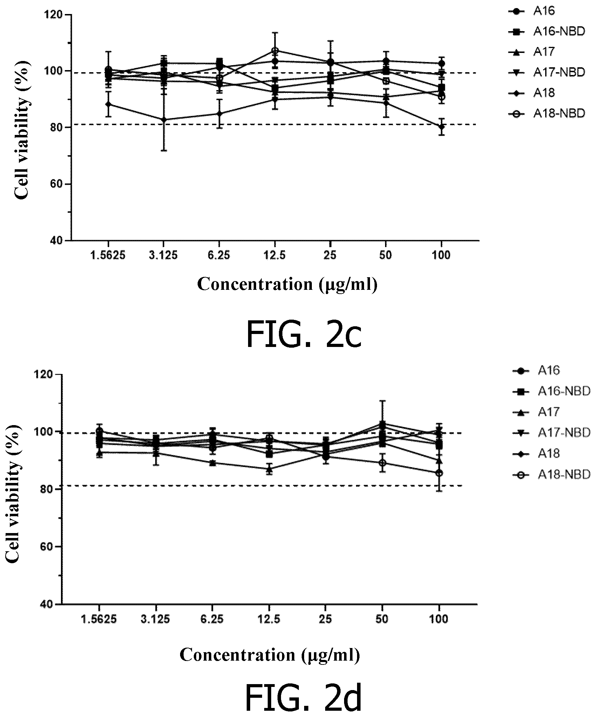

As shown in FIG. 2a - FIG. 2d, it was demonstrated that, for example, A13 represented a liposome containing dendron-like lipid compound A13, and A13-NBD represented using liposome A13 labeled with NBD 16:0 lipophilic fluorescent substance; and the other groups had the same meanings. Compared with the control group to which no drug was added, the cell viability (%) could be calculated. It could be seen that neither of PYR and 7F with different carbon chains had obvious cytotoxicity to macrophages and cancer cells within 100 µg/ml, and the cell viability could reach 80% or more.

### APPLICATION EXAMPLE 6

### Evaluation of transfection of cells with siRNA-encapsulating liposomes

eGFP-231 cells were plated on a 96-well plate with the cell number being 1 × 10⁴ cells/well and cultured at 37°C under the conditions of 95% relative humidity and 5% CO₂. After 24 hours of culturing, the theoretical value was 100 ng of siRNA-LNP added for transfection, and the medium was added to 100 µL. Lipo3000 was used as a positive control. Incubation was carried out for 48 h at 37°C under the conditions of 95% relative humidity and 5% CO₂. 100 µL of 1X hoechst 33342 living cell dye solution was added for dyeing in the dark for 10 minutes, it was then washed with PBS 2-3 times, 100 µL of a medium was added, and it was observed under an inverted fluorescence microscope. eGFR could be excited at 488 nm, and Hoechst could be excited at 346 nm. It was observed with a 10X microscope.

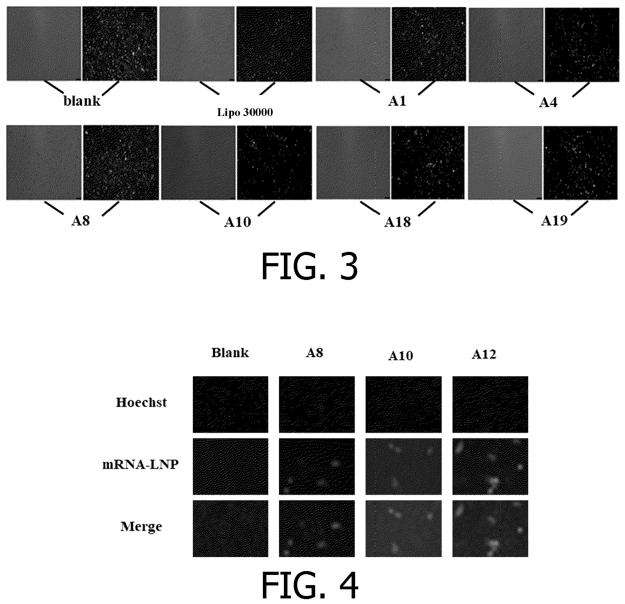

As shown in FIG. 3, it was demonstrated that, for example, the left graph in the blank group showed a bright field, and the right graph showed the fluorescence intensity of GFR. The graphs in the other groups had the same meanings. After transfection of eGFR-231 cells for 48 h, the expression of siRNA would silence the expression of eGFR, and the fluorescence intensity decreased. The transfection efficiency of siRNA could be assessed by the fluorescence intensity. As could be seen from the figure, in the experimental groups, A10 had the best effect, and A4, A18 and A19 also inhibited the expression of eGFR to some extent.

### APPLICATION EXAMPLE 7

### Evaluation of transfection of mRNA-encapsulating liposomes

A549 cells were plated on a 96-well plate with the cell number being 1 × 10⁴ cells/well and cultured at 37°C under the conditions of 95% relative humidity and 5% CO₂. After 24 hours of culturing, the theoretical value was 200 ng of eGFR mRNA-LNP added for transfection, and the medium was added to 100 µL. Lipo max was used as a positive control. Incubation was carried out for 48 h at 37°C under the conditions of 95% relative humidity and 5% CO₂. 100 µL of 1X hoechst 33342 living cell dye solution was added for dyeing in the dark for 10 minutes, it was then washed with PBS 2-3 times, 100 µL of a medium was added, and it was observed under an inverted fluorescence microscope. eGFR could be excited at 488 nm, and Hoechst could be excited at 346 nm. It was observed with a 10X microscope.

As shown in FIG. 4, it was demonstrated that, for example, in the blank group, the upper graphs were Hoechst-stained nucleus graphs, the middle graphs were the fluorescence intensity of GFR, and the lower graphs were the superposition of the two graphs. The graphs in the other group had the same meanings. After transfection of A549 cells for 48 h, eGFR mRNA was expressed, the fluorescence of eGFR was visible, and the transfection efficiency of mRNA could be assessed by fluorescence intensity. As could be seen from the figure, in the experimental groups, A12 and A10 had the best effects, and A8 could also transfect mRNA into cells to some extent.

### APPLICATION EXAMPLE 8

### Evaluation of tissue targeting of liposomes

Preparation of fluorescent liposomes: the synthesized dendron-like lipid compounds (A9 and A18), DSPC, cholesterol, DMG-PEG2000, and the lipophilic fluorescent dye Egg Liss Rhod PE were respectively dissolved in anhydrous ethanol for later use. The above solutions were mixed at a molar ratio of 50 : 10 : 34 : 5 : 1. Under stirring, the alcohol phase solution prepared above was dropwise added to PBS, with the volume ratio of alcohol phase to PBS being 1 : 3, and after mixing, the materials were stirred for 1 minute. The reaction solution was added to a 10 K MVCO ultrafiltration centrifuge tube, PBS was added, and the tube was balanced. A high-speed centrifuge was used for centrifugation at 4000 g for 25 minutes. After the solution was concentrated, PBS was added again for centrifugation at 4000 g for 25 minutes, and solution displacement was carried out to finally obtain a liposome solution.

Living imaging of animals: 6-8 weeks old Balb/c mice, half male and half female, were purchased, the mice were injected with fluorescent liposomes by tail vein injection, and living imaging was performed at 1 h, 2 h, 4 h and 24 h after tail vein injection. After deep anesthesia, the mice were sacrificed by cervical dislocation, and the brain, heart, lung, liver, spleen, kidney and gastrointestinal tract of the mice were taken for organ imaging, and the excitation wavelength and emission wavelength were set to 535 nm and 580 nm. The organ targeting of the liposomes was assessed by counting the fluorescence intensity in organs.

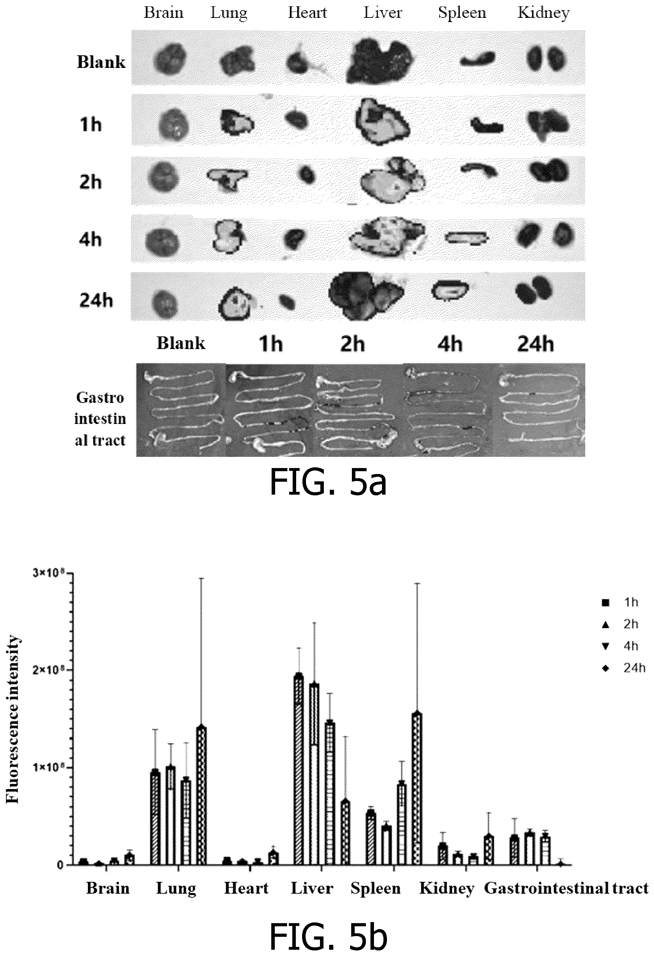

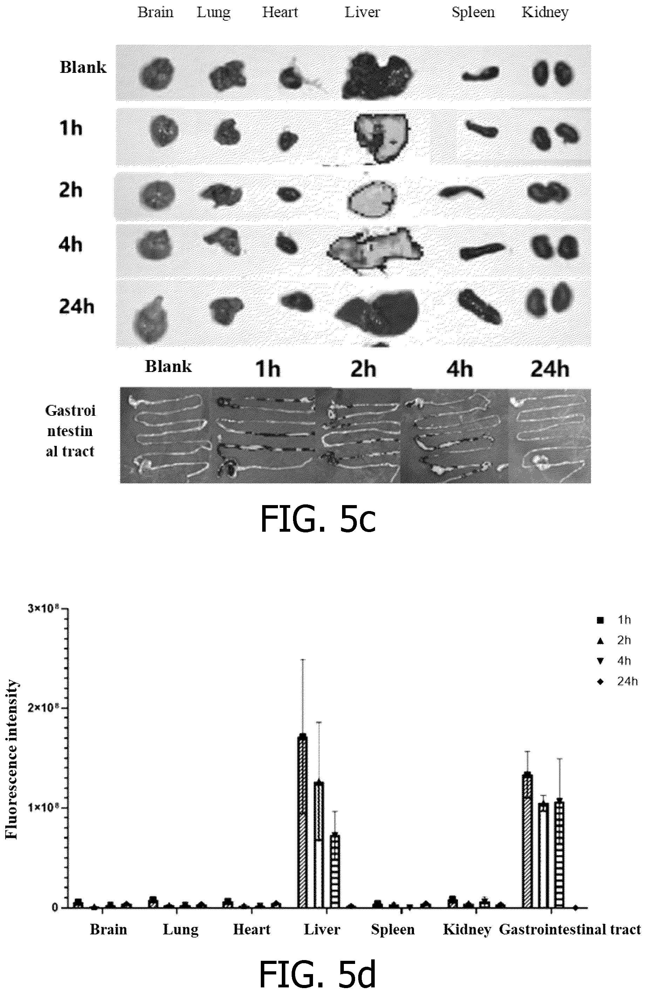

As shown in FIG. 5a - FIG. 5d, it was demonstrated that, for example, A9 represented a fluorescent liposome containing dendron-like lipid compound A9, and the blank represented the organs of mice injected by tail vein with the same volume of PBS. The other groups had the same meanings. As shown in FIG. 5a - FIG. 5b, after the fluorescent liposomes entered the body of the animals, it could be seen from organ imaging that liposome A9 mainly targeted the lung, liver and spleen, and were mainly concentrated in the liver within 2 h, and partially targeted the lung and spleen. With the passage of time, the number of liposomes that had entered the lung and spleen increased gradually, and the number of liposomes in the liver decreased. After 24 h, there was still a larger amount of liposomes distributed in the body. As shown in FIG. 5c - FIG.5d, liposome A18 mainly targeted the liver and gastrointestinal tract. With the passage of time, the fluorescence intensity gradually weakened, and it was basically metabolized within 24 h.

The applicant states that the present application illustrates the process and method of the present application through the above-mentioned examples; however, the present application is not limited to the above-mentioned process steps, that is, it does not mean that the present application can only be implemented by relying on the above process steps. It should be clear to those skilled in the technical field to which it belongs that any improvement to the present application, equivalent substitution of the raw materials selected for the present application, the addition of auxiliary components, the selection of specific methods, etc., all fall within the scope of protection and disclosure of the present application.

## Claims

1. A dendron-like lipid compound, **characterized in that** the dendron-like lipid compound is selected from any one of structures of Formula I to Formula III: 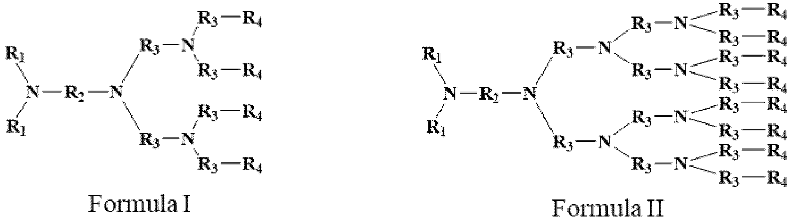 or 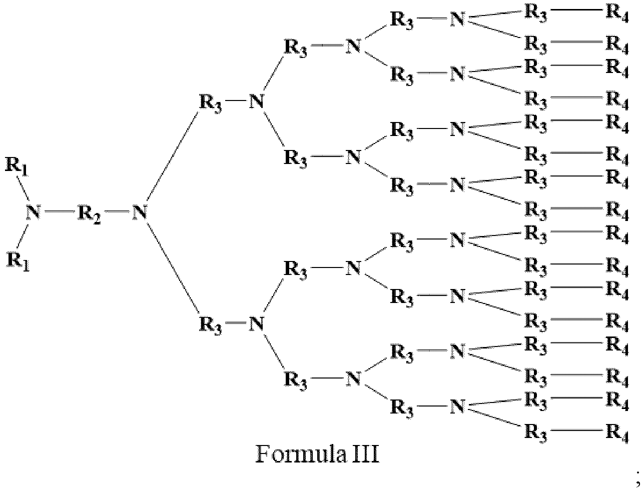
wherein Ri is selected from 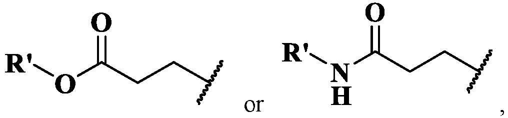 wherein R' is selected from any one of H, linear C1-C18 alkyl, or linear C2-C18 alkenyl containing 1-3 double bonds;
R₂ is selected from 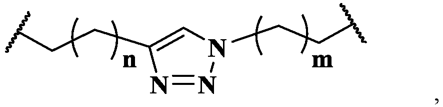 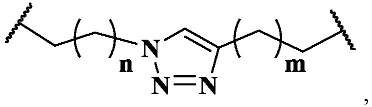 or -(CH₂)_{X}-, wherein m and n are each independently selected from an integer of 0 or between 1 and 5, and x is selected from an integer between 2 and 8;
R₃ is selected from 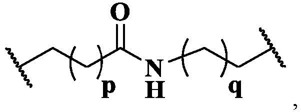 wherein p and q are each independently selected from an integer between 1 and 7;
R₄ is selected from 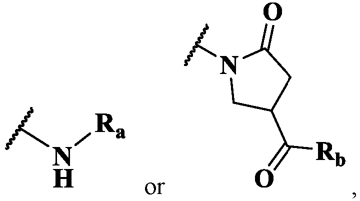 wherein Rₐ is selected from H, substituted or unsubstituted C1-C10 acyl, and substituted or unsubstituted benzoyl; R_{b} is selected from hydroxyl or C1-C10 alkoxy; and 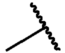
represents a site of attachment in a group.

2. The dendron-like lipid compound according to claim 1, **characterized in that** a substituent on the C1-C10 acyl and a substituent on the benzoyl are each independently selected from halogen or C1-C10 alkoxy;
preferably, Rₐ is selected from any one of H, acetyl, propionyl, n-butyryl, isobutyryl, tert-butoxyacyl, trifluoroacetyl, pentafluoropropionyl, heptafluorobutyryl, trichloroacetyl, benzoyl, 4-fluorobenzoyl, 2,3,4,5,6-pentafluorobenzoyl, 4-trifluoromethylbenzoyl, 3,4-dichlorobenzoyl, or 4-trichloromethylbenzoyl;
preferably, R_{b} is selected from any one of hydroxyl, methoxy, ethoxy, n-propoxy, isopropoxy, or n-butoxy.

3. The dendron-like lipid compound according to claim 1 or 2, **characterized in that** the dendron-like lipid compound is selected from any one of structures of Formula IV to Formula VI: 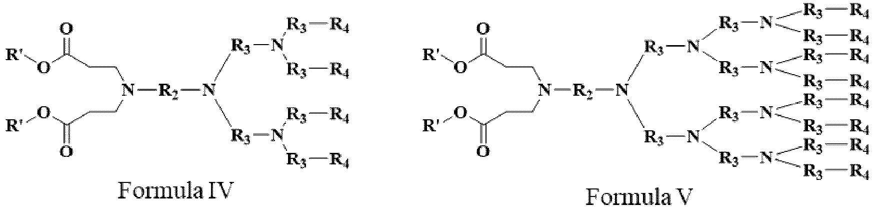 or 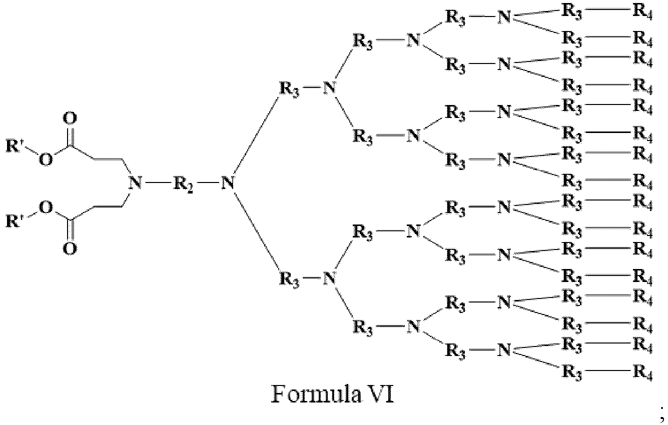
wherein R' is selected from any one of n-octyl, n-dodecyl, n-tridecyl, n-tetradecyl, n-octadecyl, or (9Z,12Z)-n-octadecadienyl, preferably any one of n-dodecyl, n-tridecyl, or (9Z, 12Z)-n-octadecadienyl;
R₂, R₃, and R₄ have the same definitions as those for R₂, R₃, and R₄ in claim 1;
preferably, the dendron-like lipid compound has any one of structures of Formula VII to Formula IX: 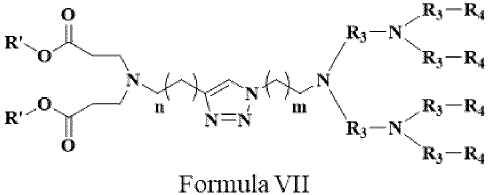 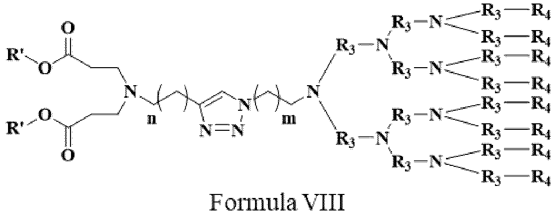 or 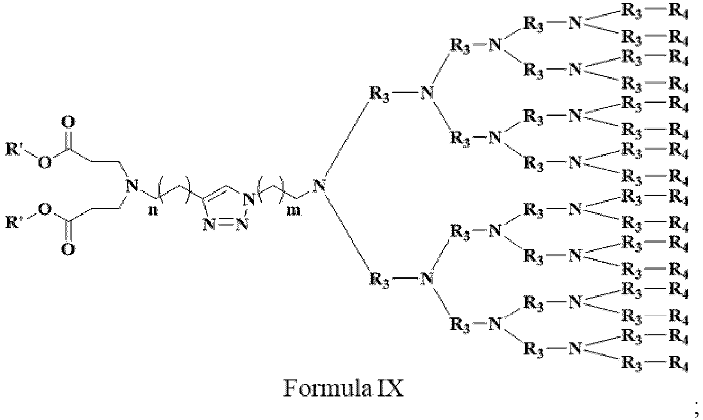
wherein R' is selected from any one of n-octyl, n-dodecyl, n-tridecyl, n-tetradecyl, n-octadecyl, or (9Z,12Z)-n-octadecadienyl, preferably any one of n-dodecyl, n-tridecyl, or (9Z,12Z)-n-octadecadienyl;
m and n are each independently selected from 0 or an integer between 1 and 5; and
R₃ and R₄ have the same definitions as those for R₃ and R₄ in claim 1.

4. The dendron-like lipid compound according to any one of claims 1-3, **characterized in that** the dendron-like lipid compound has any one of structures of Formula X to Formula XII: 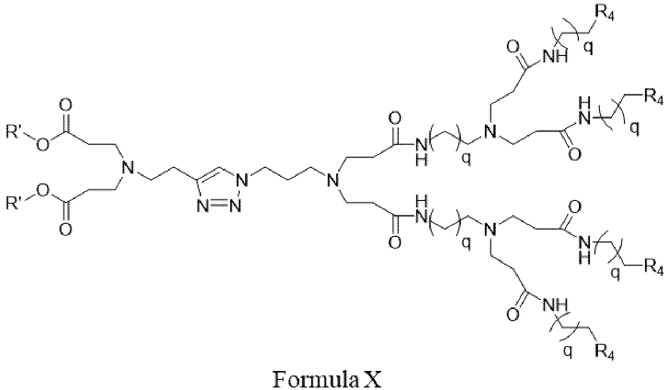 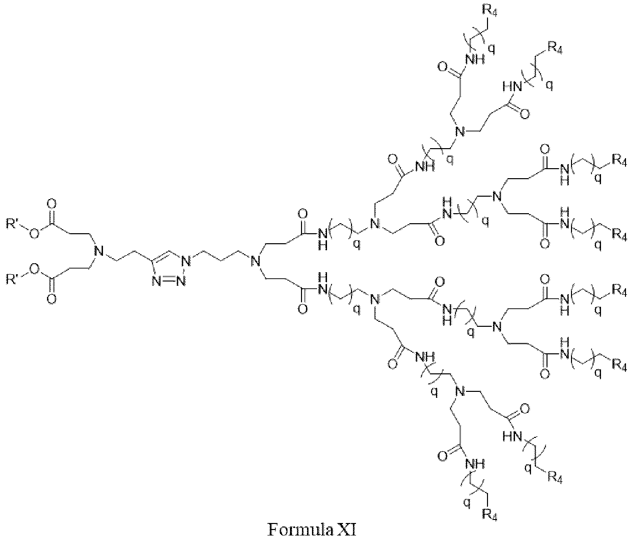 or 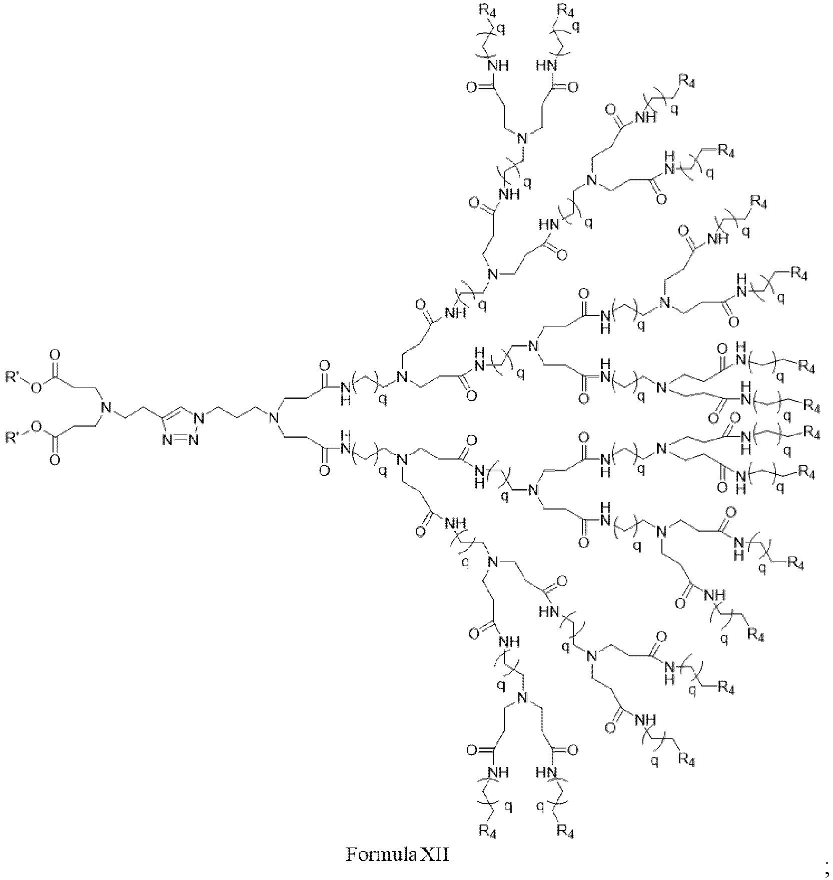
wherein R' is selected from any one of n-octyl, n-dodecyl, n-tridecyl, n-tetradecyl, n-octadecyl, or (9Z,12Z)-n-octadecadienyl, preferably any one of n-dodecyl, n-tridecyl, or (9Z,12Z)-n-octadecadienyl;
q is an integer selected between 1 and 7; and
R₄ has the same definition as that for R₄ in claim 1;
preferably, the dendron-like lipid compound has any one of structures of Formula XIII to Formula XV: 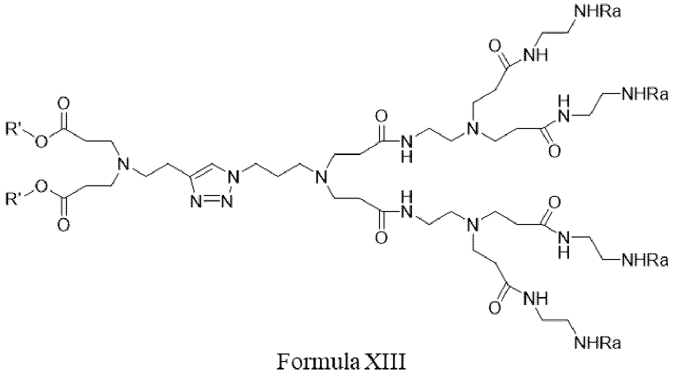 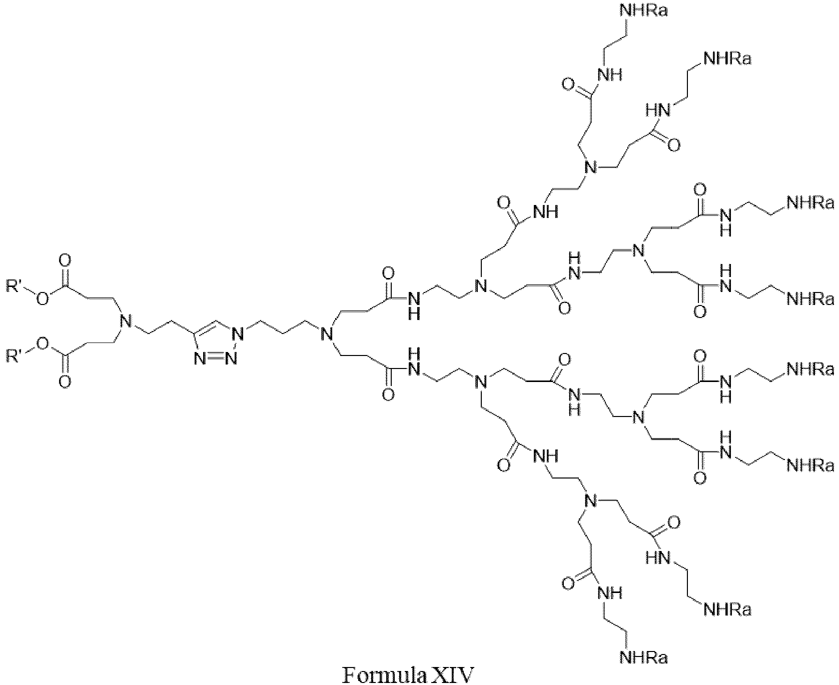 or 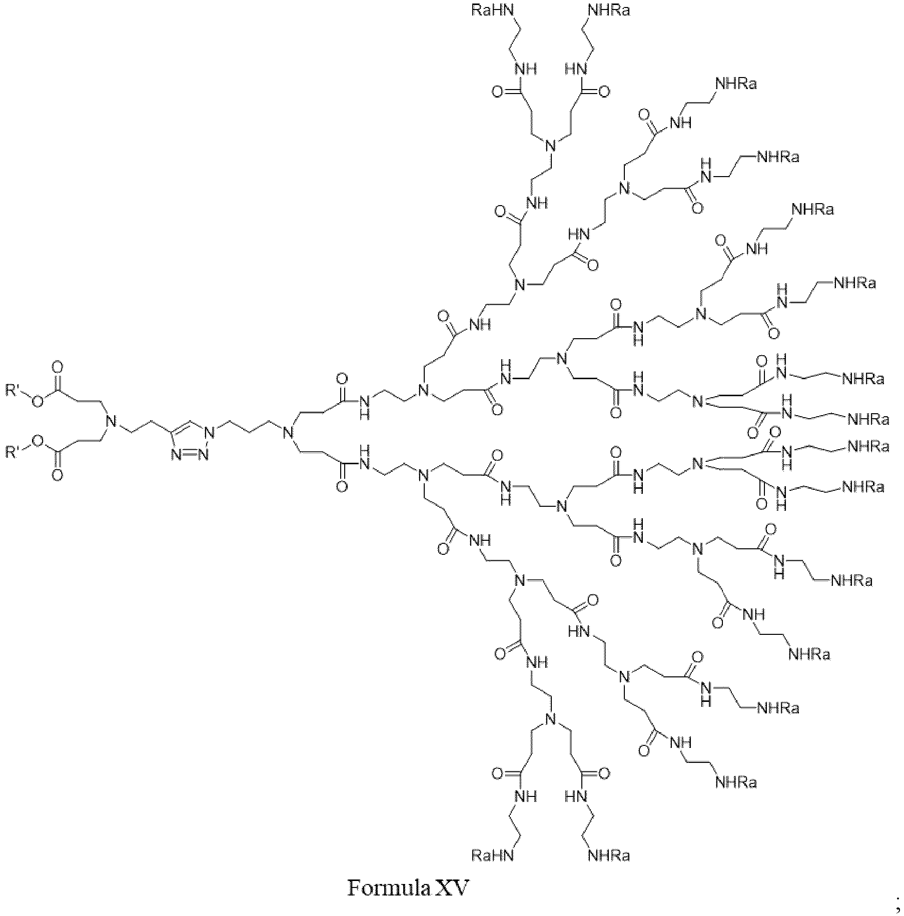
wherein R' is selected from any one of n-octyl, n-dodecyl, n-tridecyl, n-tetradecyl, n-octadecyl, or (9Z,12Z)-n-octadecadienyl, preferably any one of n-dodecyl, n-tridecyl, or (9Z, 12Z)-n-octadecadienyl;
Ra is selected from any one of H, acetyl, propionyl, n-butyryl, isobutyryl, tert-butoxyacyl, trifluoroacetyl, pentafluoropropionyl, heptafluorobutyryl, trichloroacetyl, benzoyl, 4-fluorobenzoyl, 2,3,4,5,6-pentafluorobenzoyl, 4-trifluoromethylbenzoyl, 3,4-dichlorobenzoyl, or 4-trichloromethylbenzoyl; preferably any one of H, tert-butoxyacyl, trifluoroacetyl, pentafluoropropionyl, or heptafluorobutyryl;
preferably, the dendron-like lipid compound is selected from any one of structures of Formula XVI to Formula XVIII: 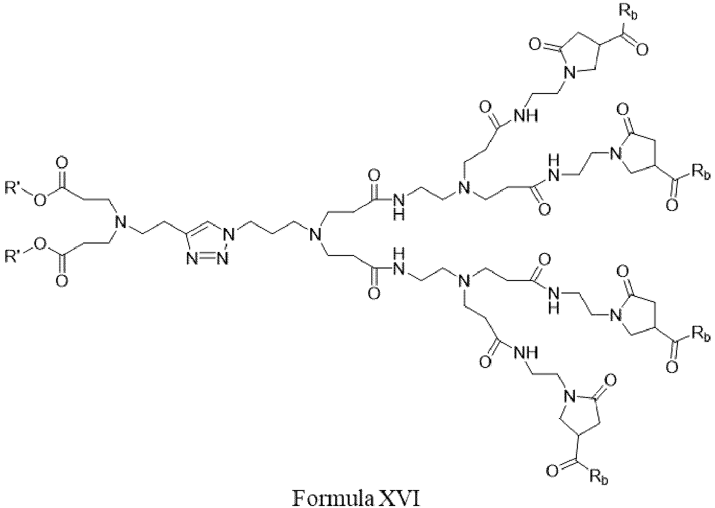 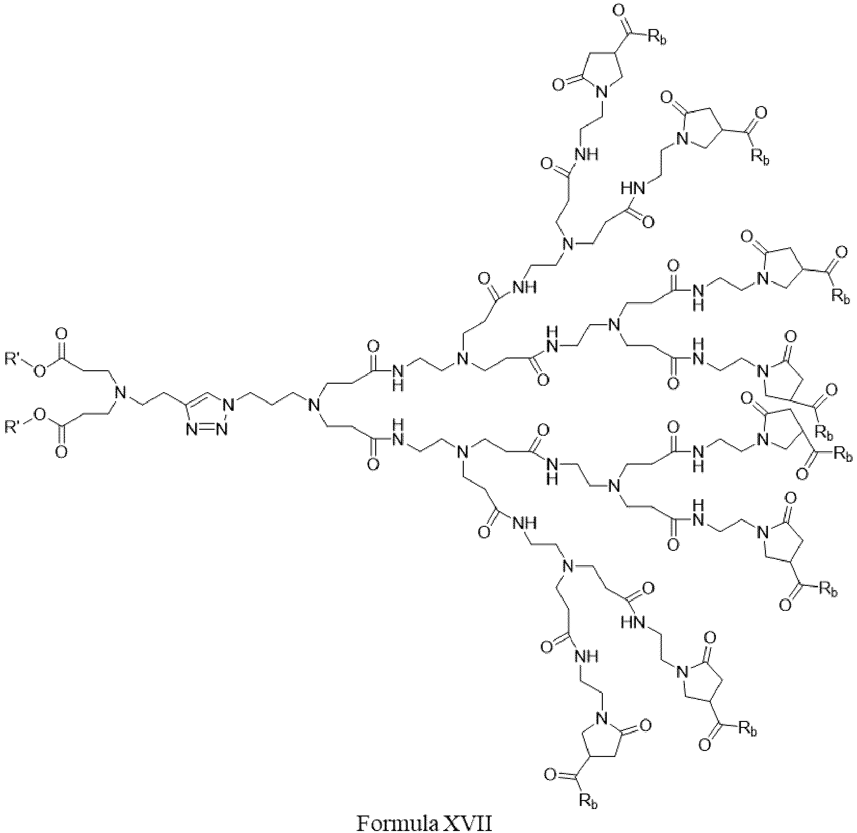 or 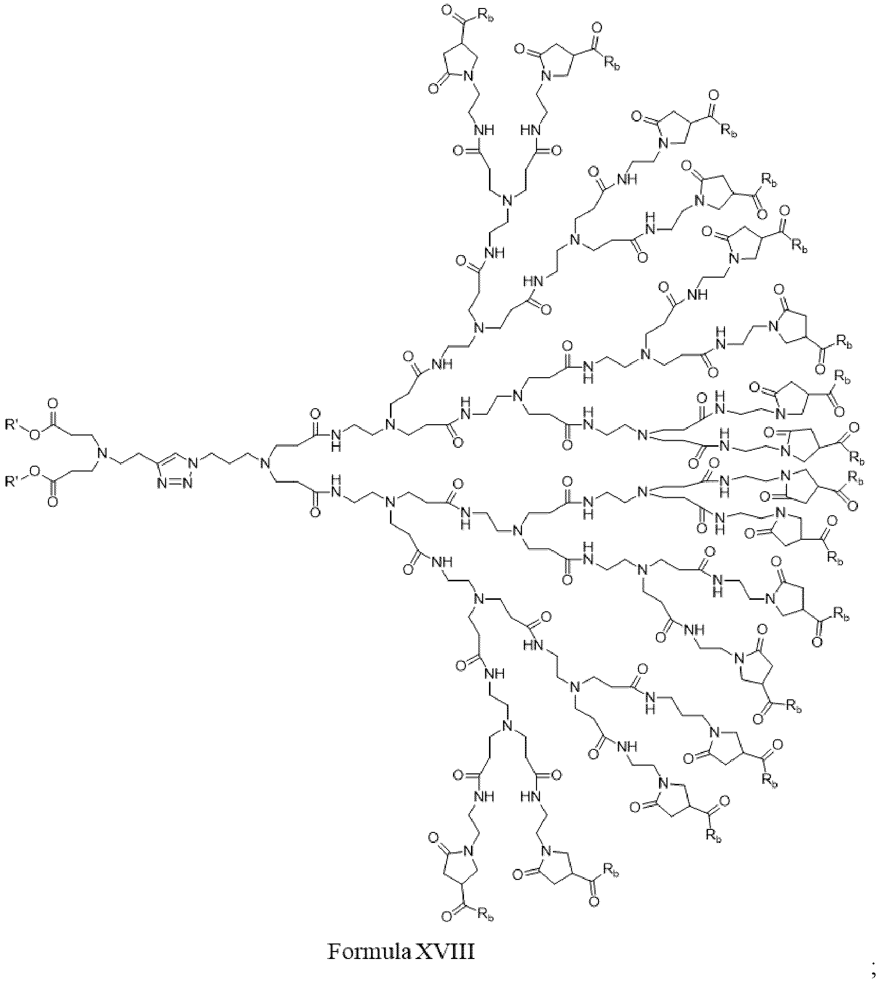
wherein R' is selected from any one of n-octyl, n-dodecyl, n-tridecyl, n-tetradecyl, n-octadecyl, or (9Z,12Z)-n-octadecadienyl, preferably any one of n-dodecyl, n-tridecyl, or (9Z,12Z)-n-octadecadienyl;
R_{b} is selected from any one of hydroxyl, methoxy, ethoxy, n-propoxy, isopropoxy, or n-butoxy, preferably methoxy.

5. The dendron-like lipid compound according to any one of claims 1-4, **characterized in that** the dendron-like lipid compound is selected from any one of the following compounds A1-A21: 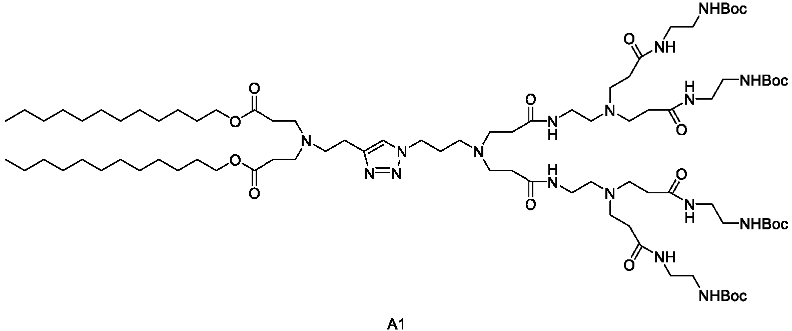 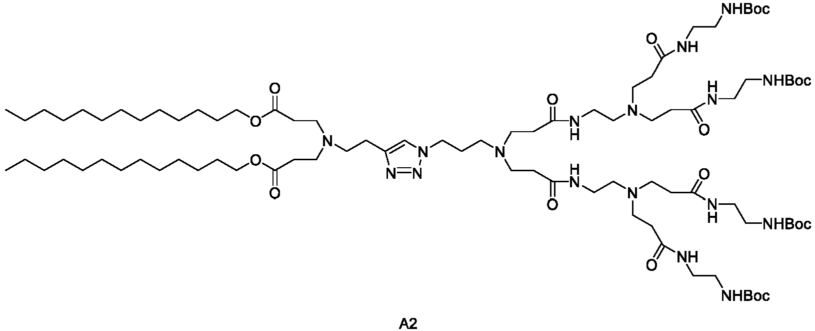 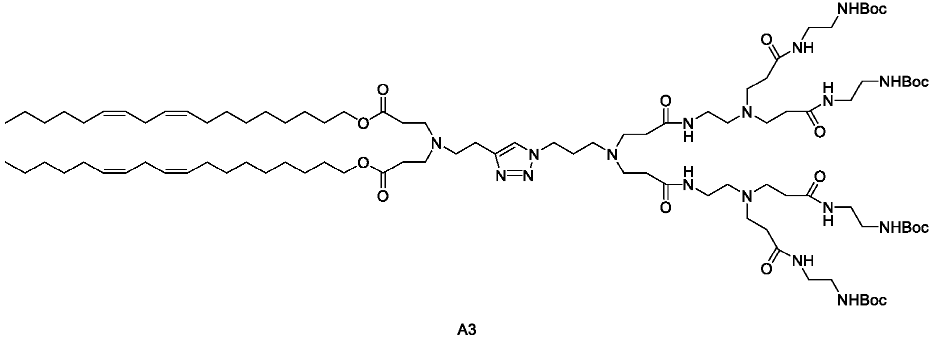 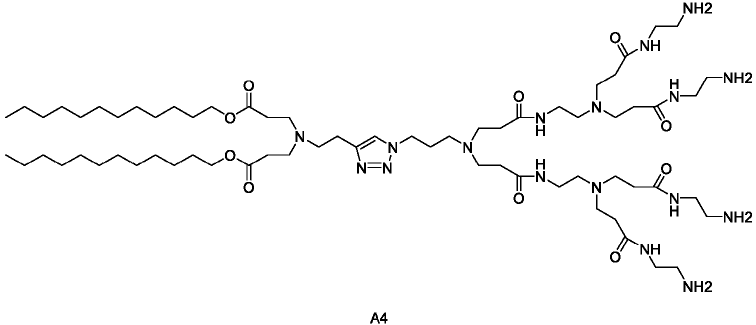 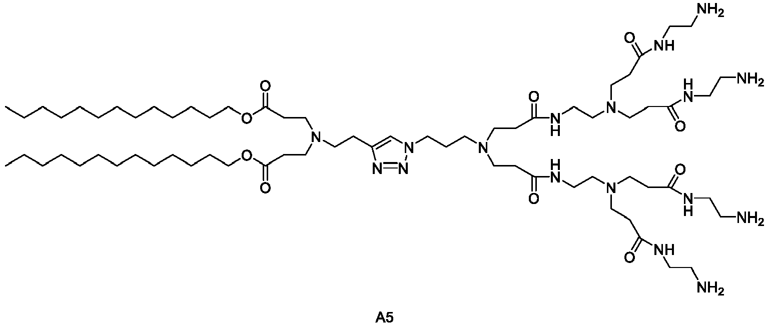 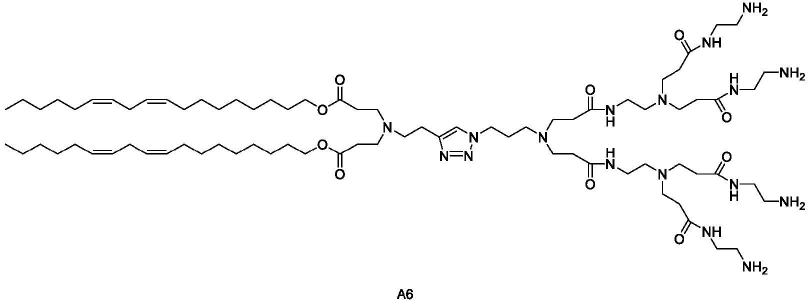 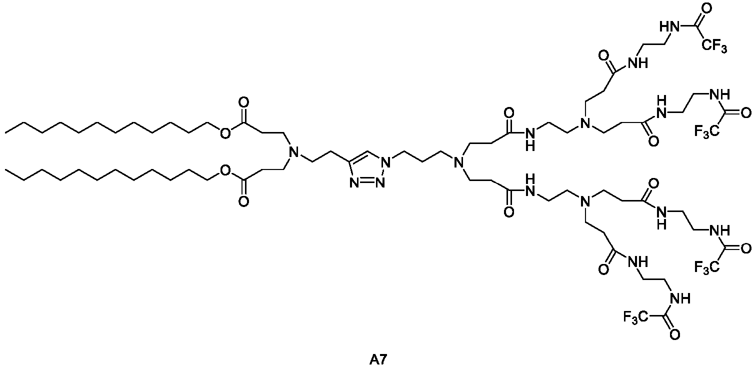 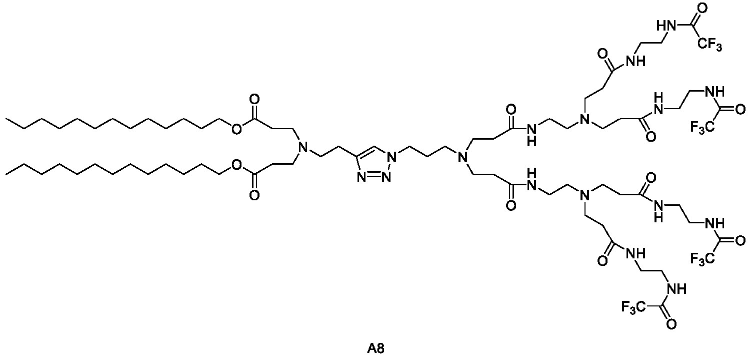 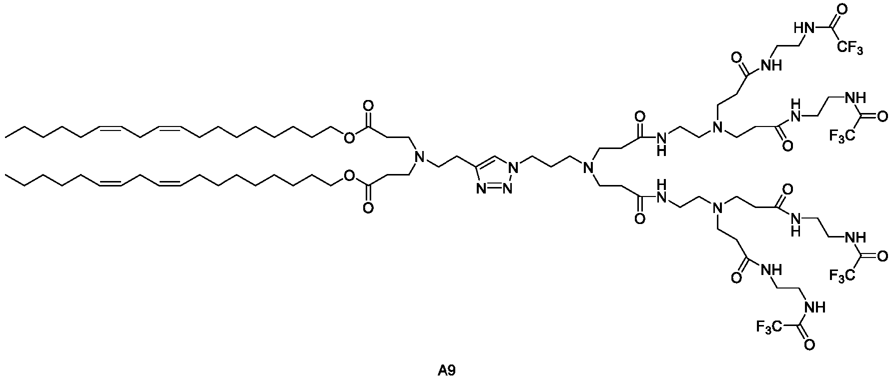 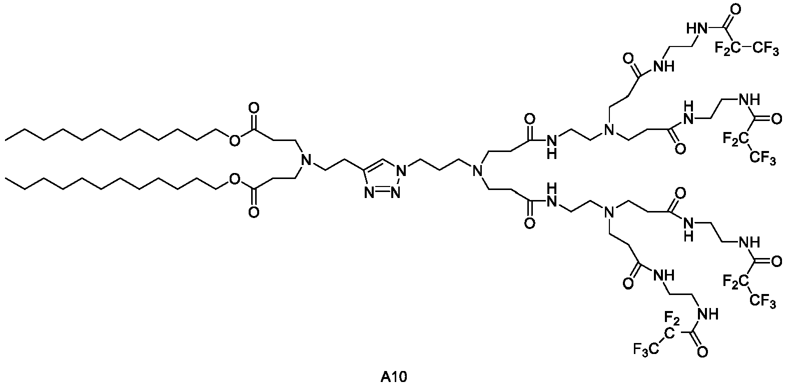 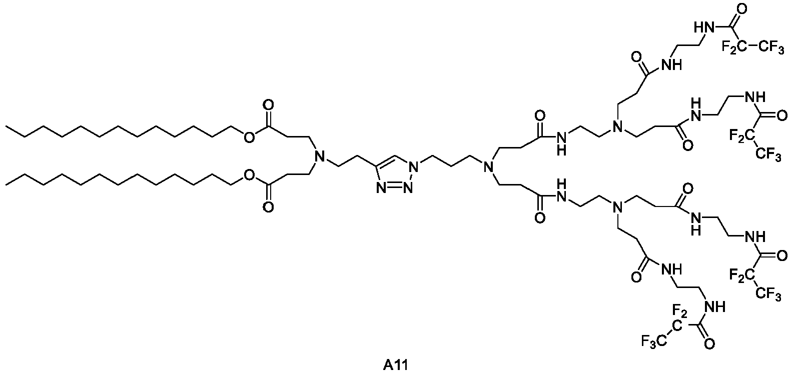 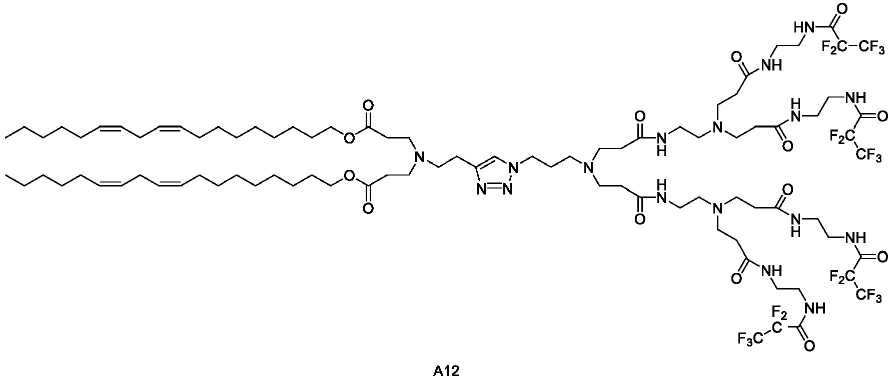 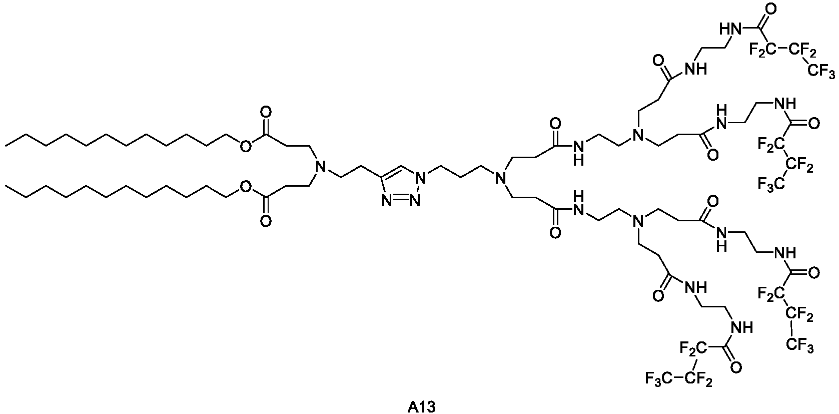 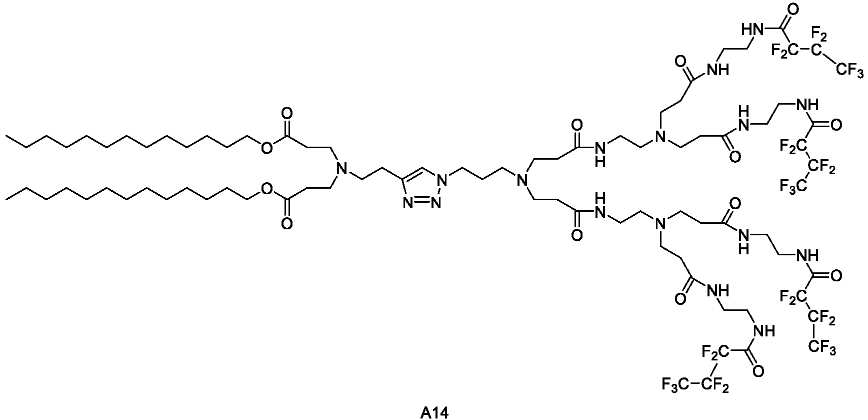 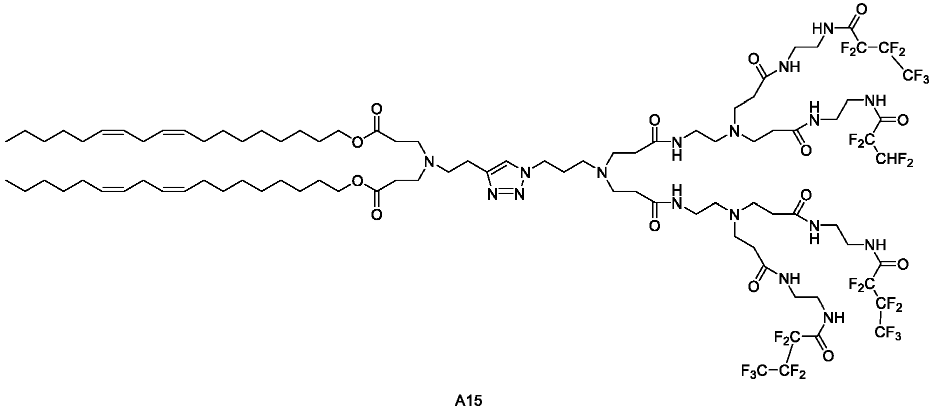 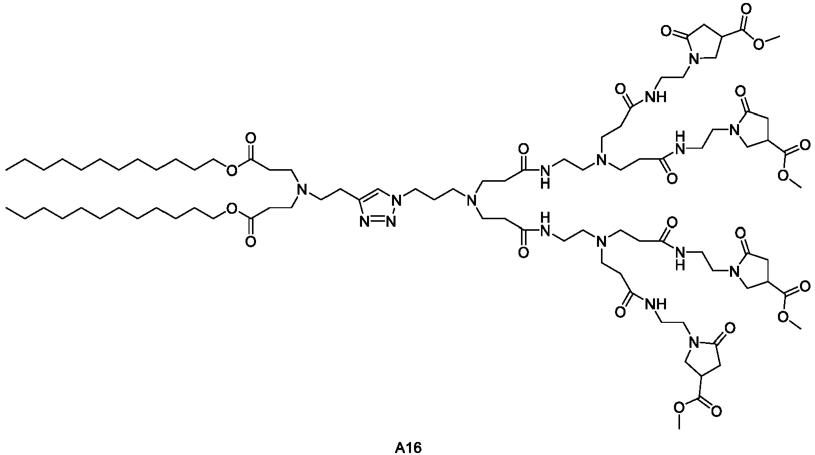 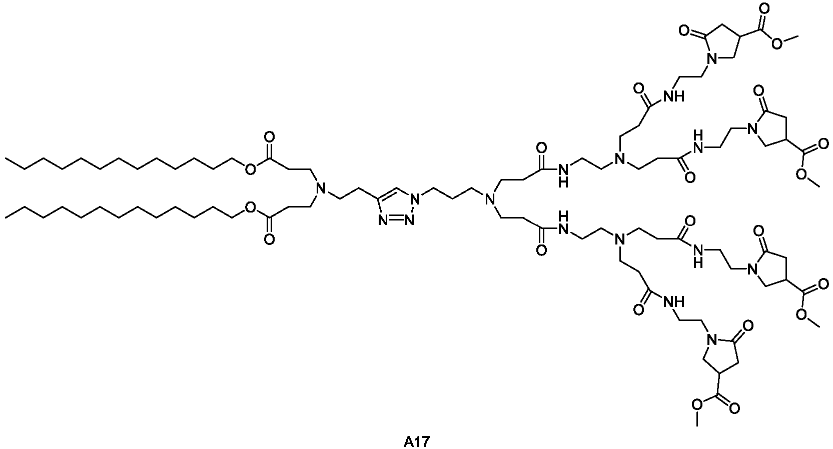 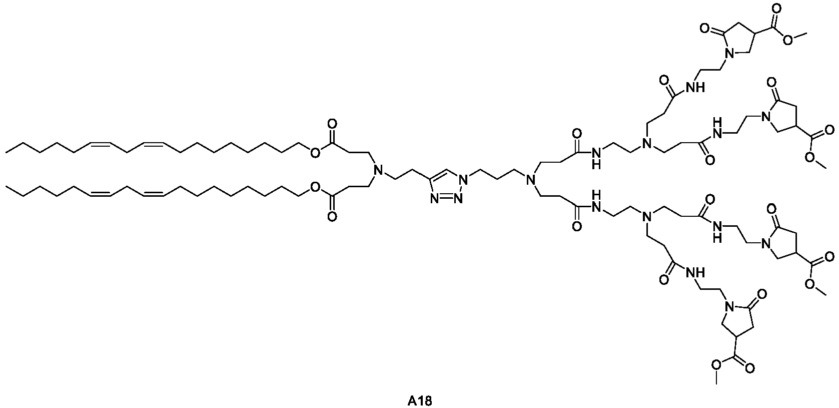 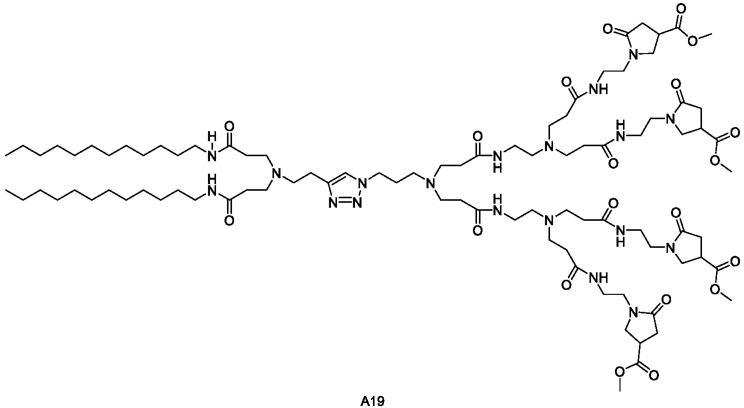 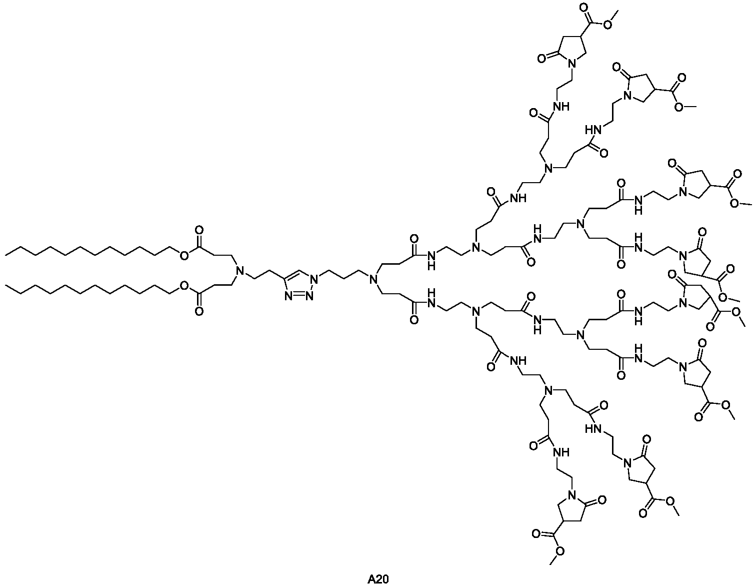 or

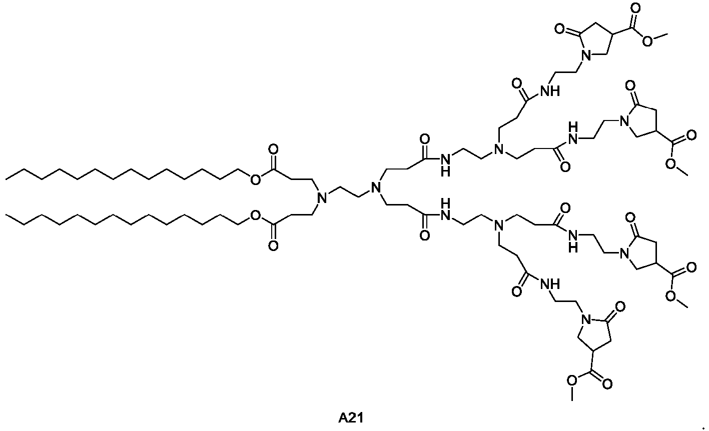

6. A liposome comprising the dendron-like lipid compound according to any one of claims 1-5, **characterized in that**
preferably, the liposome further comprises an auxiliary lipid;
preferably, the auxiliary lipid comprises a phospholipid;
preferably, the phosphatide comprises any one of or a combination of at least two of 1,2-dilinoleoyl-sn-glycero-3-phosphocholine, 1,2-dimyristoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphocholine, 1,2-diundecanoyl-sn-glycero-3-phosphocholine, 1-palmitoyl-2-oleoyl-sn-glycero-3-phosphocholine, 1,2-di-O-octadecenyl-sn-glycero-3-phosphocholine, 1-oleoyl-2-cholesterylhemisuccinoyl-sn-glycero-3-phosphocholine, 1-cetyl-sn-glycero-3-phosphocholine, 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine, 1,2-diphytanyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt, dipalmitoylphosphatidylglycerol, palmitoyloleoylphosphatidylethanolamine, distearoyl-phosphatidyl-ethanolamine, dipalmitoylphosphatidylethanolamine, dimyristoylphosphoethanolamine, 1-stearoyl-2-oleoyl-stearoylethanolamine, 1-stearoyl-2-oleoyl-phosphatidylcholine, sphingomyelin, phosphatidylcholine, phosphatidylethanolamine, phosphatidylserine, phosphatidylinositol, phosphatidic acid, palmitoyloleoylphosphatidylcholine, lysophosphatidylcholine, or lysophosphatidylethanolamine;
preferably, the auxiliary lipid further comprises a structured lipid;
preferably, the structured lipid comprises at least any at least one of or a combination of at least two of cholesterol, coprosterol, sitosterol, ergosterol, rapeseed sterol, soybean sterol, rapeseed sterol, stigmasterol, brassicasterol, tomatidine, ursolic acid, or α-tocopherol;
preferably, the auxiliary lipid further comprises a PEGylated lipid;
preferably, the PEGylated lipid comprises any one of or a combination of at least two of PEG-modified phosphatidylethanolamine, PEG-modified phosphatidic acid, PEG-modified ceramide, or PEG-modified dialkylamine;
preferably, the liposome comprises a dendron-like lipid compound, a phospholipid, a structured lipid, and a PEGylated lipid;
preferably, the liposome comprises, by mole percentage, 20%-80% of a dendron-like lipid compound, 5%-30% of a phospholipid, 10%-50% of a structured lipid, and 1%-10% of a PEGylated lipid.

7. A lipid complex comprising the liposome according to claim 6 and an active substance, **characterized in that**
preferably, the active substance comprises any one of or a combination of at least two of a small molecule compound, a nucleic acid molecule, or a protein molecule;
preferably, the nucleic acid molecule comprises any at least one of or a combination of at least two of DNA, mRNA, siRNA, aiRNA, miRNA, dsRNA, aRNA, or IncRNA;
preferably, the protein molecule comprises any one of or a combination of at least two of an enzyme, an antibody, a short peptide, a polypeptide, or a recombinant protein;
preferably, a method for preparing the lipid complex comprises a step of mixing a liposome with an active substance by means of a mixer to obtain the lipid complex.

8. A lipid nanoparticle comprising the liposome according to claim 6 or the lipid complex according to claim 7, **characterized in that**
preferably, the particle size of the lipid nanoparticle is 40 nm-300 nm.

9. A reagent, kit, preparation or pharmaceutical composition, **characterized in that** each of which independently comprises any one of or a combination of at least two of the dendron-like lipid compound according to any one of claims 1-5, the liposome according to claim 6, the lipid complex according to claim 7, or the lipid nanoparticle according to claim 8.

10. Use of the dendron-like lipid compound according to any one of claims 1-5, the liposome according to claim 6, the lipid complex according to claim 7, the lipid nanoparticle according to claim 8, or the reagent, kit, preparation or pharmaceutical composition according to claim 9 in the manufacture of a product with any one of the following functions (1) to (4):
(1) encapsulating an active substance;
(2) delivering an encapsulated active substance to a cell, a tissue, or an organ;
(3) enabling a delivered active substance to function in a cell, a tissue, or an organ; and
(4) preventing, diagnosing, or treating a disease.
